(19) Europäisches Patentamt
European Patent Office
Office européen des brevets

(11) Veröffentlichungsnummer: **0 310 737 A1**

(12) **EUROPÄISCHE PATENTANMELDUNG**

(21) Anmeldenummer: **88102619.9**

(22) Anmeldetag: **23.02.88**

(51) Int. Cl.⁴: **C07D 403/04 , A61K 31/415 , A61K 31/50**

Patentansprüche für folgende Vertragsstaaten:GR + ES.

(30) Priorität: **08.10.87 DE 3734083**

(43) Veröffentlichungstag der Anmeldung: **12.04.89 Patentblatt 89/15**

(84) Benannte Vertragsstaaten: **AT BE CH DE ES FR GB GR IT LI LU NL SE**

(71) Anmelder: **HEUMANN PHARMA GMBH & CO**
**18-28 Heideloffstrasse**
**D-8500 Nürnberg(DE)**

(72) Erfinder: **Mörsdorf, Peter Dr.**
**Ziegelstrasse 64**
**D-8506 Langenzehn(DE)**
Erfinder: **Schickaneder, Helmut Dr.**
**Moosäcker 25**
**D-8501 Eckental(DE)**
Erfinder: **Herter, Rolf Dr.**
**Bayernstrasse 42**
**D-8540 Schwabach(DE)**
Erfinder: **Pfahlert, Volker Dr.**
**Hummelsteinerweg 75**
**D-8500 Nürnberg(DE)**
Erfinder: **Engler, Heidrun Dr.**
**Ringstrasse 23**
**D-8501 Cadolzburg(DE)**
Erfinder: **Ahrens, Kurt Henning Dr.**
**Praterstrasse 9**
**D-8500 Nürnberg(DE)**

(74) Vertreter: **Kraus, Walter, Dr. et al**
**Patentanwälte Kraus, Weisert & Partner**
**Thomas-Wimmer-Ring 15**
**D-8000 München 22(DE)**

(54) **Benzimidazole, Verfahren zu ihrer Herstellung und diese Verbindungen enthaltende Arzneimittel.**

(57) Es werden neue Benzimidazole der allgemeinen Formel I

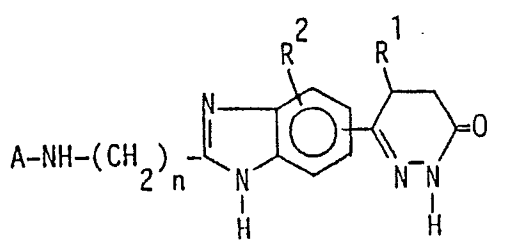

(I)

in denen der Pyridazinonring in 5- oder 6-Stellung des Benzimidazolrings gebunden ist und $R^1$ ein Wasserstoffatom oder eine gerad- oder verzweigtkettige $C_1$-$C_4$-Alkylgruppe bedeutet, $R^2$ für ein Wasser-

**EP 0 310 737 A1**

stoffatom, eine gerad- oder verzweigtkettige $C_1$-$C_4$- Alkylgruppe, eine $C_1$-$C_4$-Alkoxygruppe. eine Hydroxygruppe, ein Halogenatom, eine Aminogruppe oder eine Nitrogruppe steht, A für ein Wasserstoffatom, die Gruppe 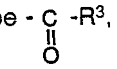

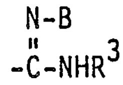 oder 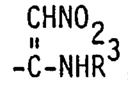,

worin $R^3$ ein Wasserstoffatom, eine gegebenenfalls substituierte $C_1$-$C_4$- Alkylgruppe oder eine $C_1$-$C_4$-Alkoxygruppe und B eine Cyanogruppe, eine Benzoylgruppe oder eine Phenylsulfonylgruppe bedeuten, oder für die Gruppierung

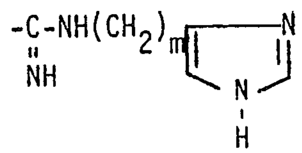 ,

in der m den Wert 2 oder 3 hat, steht und n eine ganze Zahl von 1 bis 6 ist, sowie die physiologisch annehmbaren Salze davon, Verfahren zu ihrer Herstellung und diese Verbindungen enthaltende Arzneimittel beschrieben. Diese Verbindungen sind neue wirksame, positiv inotrope Substanzen, die nicht über den Mechanismus der Phosphodiesterase-Hemmung wirken.

Die medikamentöse Behandlung der Herzinsuffizienz beruhte fast 200 Jahre auf der Verwendung von Digitalisglycosiden, wie Digoxin und Digitoxin, da diese Substanzen die einzigen mit inotropiesteigernder Wirkung waren und damit die einzige Möglichkeit, die Ursache des Herzversagens, nämlich die mangelnde Kontraktilität des Herzens, zu bessern.

Die Suche nach Digitalis-Ersatzsubstanzen führte zunächst zu den Sympathomimetika, die jedoch eine Reihe von gewichtigen Nachteilen, wie unerwünschte chronotrope und arrhythmogene Nebenwirkungen und fehlende orale Verfügbarkeit aufweisen. In den letzten zehn Jahren wurde dann in schneller Folge eine Reihe von Substanzen gefunden, die über eine Hemmung der Phosphodiesterasen der Herzmuskelzelle, insbesondere der Phosphodiesterase, Typ III (PDE III) eine positiv inotrope, d.h. kontraktilitätssteigernde Wirkung hervorrufen. Beispiele für diese PDE-Hemmer sind Amrinon (J.R. Benotti u.a., N. Engl. J. Med. 299, 1373 (1978)), Milrinon (A.A. Alousi u.a., J. Cardiovasc. Pharmacol. 5, 792 (1983)) und das Benzimidazolderivat Pimobendan (J.C.A. von Meel, Arzneim-Forsch. 35, 284 (1985)). Jedoch haben auch diese Substanzen aufgrund ihrer Wirkungsmechanismen unerwünschte Nebenwirkungen, wie Thrombocytopenie oder gastrointestinale Störungen.

Der Erfindung liegt deshalb die Aufgabe zugrunde, neue wirksame, positiv inotrope Substanzen zu finden, die nicht über den Mechanismus der Phosphodiesterase-Hemmung wirken.

Diese Aufgabe wird durch die Erfindung gelöst.

Gegenstand der Erfindung sind Benzimidazole der allgemeinen Formel I

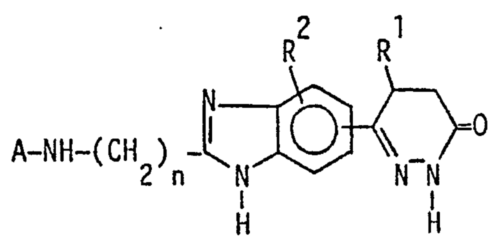

(I)

in denen der Pyridazinonring in 5- oder 6-Stellung des Benzimidazolrings gebunden ist und $R^1$ ein Wasserstoffatom oder eine gerad- oder verzweigtkettige $C_1$-$C_4$-Alkylgruppe bedeutet, $R^2$ für ein Wasserstoffatom, eine gerad- oder verzweigtkettige $C_1$-$C_4$-Alkylgruppe, eine $C_1$-$C_4$-Alkoxygruppe, eine Hydroxygruppe, ein Halogenatom, eine Aminogruppe oder eine Nitrogruppe steht, A für ein Wasserstoffatom, die Gruppe $-\underset{\underset{O}{\|}}{C}-R^3$,

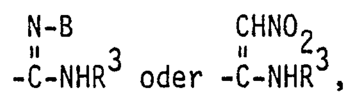

worin $R^3$ ein Wasserstoffatom, eine gegebenenfalls substituierte $C_1$-$C_4$-Alkylgruppe oder eine $C_1$-$C_4$ Alkoxygruppe und B eine Cyanogruppe, eine Benzoylgruppe oder eine Phenylsulfonylgruppe bedeuten, oder für die Gruppierung

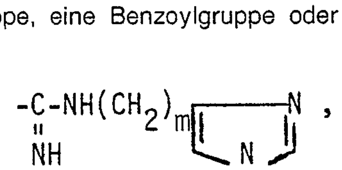

in der m den Wert 2 oder 3 hat, steht und n eine ganze Zahl von 1 bis 6 ist, sowie die physiologisch annehmbaren Salze davon.

In der allgemeinen Formel I bedeutet $R^1$ ein Wasserstoffatom oder eine gerad- oder verzweigtkettige $C_1$-$C_4$-Alkylgruppe. Beispiele für solche gerad-oder verzweigtkettigen $C_1$-$C_4$-Alkylgruppen sind die Methyl-, Ethyl-, n-Propyl-, Isopropyl-, n-Butyl- und sec.-Butylgruppe, wobei die Methylgruppe bevorzugt wird. $R^2$ steht für ein Wasserstoffatom, eine gerad- oder verzweigtkettige $C_1$-$C_4$ Alkylgruppe, wie oben im Zusammenhang mit $R^1$ definiert, eine $C_1$-$C_4$-Alkoxygruppe, beispielsweise eine Methoxy-, Ethoxy-, n-Propoxy-,

Isopropoxy- oder n-Butoxygruppe, eine Hydroxygruppe, ein Halogenatom, beispielsweise ein Fluor-, Chlor- oder Bromatom, eine Aminogruppe oder eine Nitrogruppe. Steht $R^2$ für ein Halogenatom, dann wird das Chloratom bevorzugt. Besonders bevorzugt werden Verbindungen, bei denen $R^2$ für ein Wasserstoffatom steht.

A bedeutet ein Wasserstoffatom oder die Gruppe $-\underset{\underset{O}{\|}}{C}-R^3$,

worin $R^3$ für ein Wasserstoffatom, eine gegebenenfalls substituierte $C_1$-$C_4$-Alkylgruppe, beispielsweise eine Methyl-, Ethyl-, n-Propyl-, Isopropyl- oder n-Butylgruppe, oder eine $C_1$-$C_4$ Alkoxygruppe steht. Im Falle der Substitution wird die Ein- oder Zweifachsubstitution mit einem Halogenatom, zum Beispiel einem Chlor-, Brom- oder Jodatom, einer $C_1$-$C_4$-Alkoxygruppe, beispielsweise einer Ethoxy- oder Methoxygruppe, und/oder einem Arylrest, vorzugsweise einem Phenylrest, bevorzugt. Besonders bevorzugt wird aber eine unsubstituierte $C_1$-$C_4$ Alkylgruppe und insbesondere die Methylgruppe.

A kann weiterhin die Gruppierung

$$-\overset{\overset{N-B}{\|}}{C}-NHR^3$$

bedeuten. Darin hat $R^3$ die gleiche Definition wie oben angegeben. B steht für eine Cyanogruppe, eine Benzoylgruppe oder eine Phenylsulfonylgruppe, vorzugsweise für eine Cyanogruppe.

Weiterhin kann A die Gruppierung

$$-\overset{\overset{CHNO_2}{\|}}{C}-NHR^3$$

bedeuten, worin $R^3$ wiederum die oben angegebene Definition hat.

Schließlich kann A noch die Gruppierung

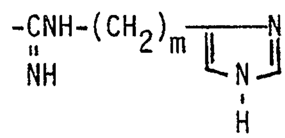

bedeuten, worin m den Wert 2 oder 3, vorzugsweise den Wert 3, hat.

n ist in jedem Fall eine ganze Zahl von 1 bis 6, vorzugsweise 2, 3 oder 4. Verbindungen, bei denen n den Wert 3 hat, werden ganz besonders bevorzugt.

Eine bevorzugte Verbindungsgruppe gemäß der vorliegenden Erfindung ist dadurch gekennzeichnet, daß in der allgemeinen Formel I $R^1$ für ein Wasserstoffatom oder eine gerad- oder verzweigtkettige $C_1$-$C_4$-Alkylgruppe, insbesondere eine Methylgruppe, steht, $R^2$ und A jeweils für ein Wasserstoffatom stehen und n eine ganze Zahl von 1 bis 6, insbesondere die Zahl 3, ist.

Eine weitere bevorzugte Verbindungsgruppe gemäß der vorliegenden Erfindung ist dadurch gekennzeichnet, daß in der allgemeinen Formel I $R^1$ für ein Wasserstoffatom oder eine gerad- oder verzweigtkettige $C_1$-$C_4$- Alkylgruppe, insbesondere eine Methylgruppe, steht, $R^2$ ein Wasserstoffatom bedeutet, A für die Gruppe $-\underset{\underset{O}{\|}}{C}-R^3$ steht,

wobei $R^3$ ein Wasserstoffatom, eine gegebenenfalls substituierte $C_1$-$C_4$-Alkylgruppe oder eine $C_1$-$C_4$ Alkoxygruppe ist, und n eine ganze Zahl von 1 bis 6, insbesondere die Zahl 3, ist.

Eine weitere bevorzugte Verbindungsgruppe gemäß der vorliegenden Erfindung ist dadurch gekennzeichnet, daß in der allgemeinen Formel I $R^1$ für ein Wasserstoffatom oder eine gerad- oder verzweigtkettige $C_1$-$C_4$-Alkylgruppe, insbesondere eine Methylgruppe, steht, $R^2$ ein Wasserstoffatom ist, A für die Gruppe

$$-\overset{\overset{N-B}{\|}}{C}-NHR^3$$

steht, worin $R^3$ ein Wasserstoffatom oder eine gegebenenfalls substituierte $C_1$-$C_4$-Alkylgruppe ist und B eine Cyanogruppe, eine Benzoylgruppe oder eine Phenylsulfonylgruppe bedeutet, und n eine ganze Zahl von 1 bis 6, insbesondere die Zahl 3, ist.

Eine weitere bevorzugte Verbindungsgruppe gemäß der vorliegenden Erfindung ist dadurch gekennzeichnet, daß $R^1$ für ein Wasserstoffatom oder eine gerad- oder verzweigtkettige $C_1$-$C_4$-Alkylgruppe, insbesondere eine Methylgruppe, und $R^2$ für ein Wasserstoffatom stehen, A die Gruppe

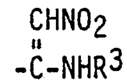

bedeutet, worin $R^3$ für ein Wasserstoffatom oder eine gegebenenfalls substituierte $C_1$-$C_4$-Alkylgruppe steht, und n eine ganze Zahl von 1 bis 6, vorzugsweise die Zahl 3, ist.

Schließlich ist eine weitere bevorzugte Verbindungsgruppe gemäß der vorliegenden Erfindung dadurch gekennzeichnet, daß in der allgemeinen Formel I $R^1$ für ein Wasserstoffatom oder eine gerad- oder verzweigtkettige $C_1$-$C_4$-Alkylgruppe, insbesondere eine Methylgruppe, steht, $R^2$ für ein Wasserstoffatom steht, A für die Gruppierung

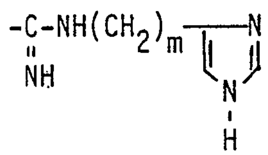 steht,

in der m den Wert 2 oder 3 hat, und n eine ganze Zahl von 1 bis 6, insbesondere die Zahl 3, ist.

Bevorzugte Einzelverbindungen sind die folgenden Verbindungen: 6-[2-(3-Aminopropyl)-1H-benzimidazol-5-yl]-4,5-dihydro-5-methyl-3(2H)-pyridazinon; $N^1$-[3-(Imidazol-4-yl)propyl]-$N^2$-[3-[5-(4-methyl-6-oxo-1,4,5,6-tetrahydropyridazin-3-yl)-1H-benzimidazol-2-yl]propyl] -guanidin; 6-|2-(2-Ami noethyl)-1H-benzimidazol-5-yl]-4,5-dihydro-5-methyl-3(2H)-pyridazinon; $N^1$-[3-(Imidazol-4-yl)propyl]-$N^2$-[2-[5-(4-methyl-6-oxo-1,4,5,6-tetrahydropyridazin-3-yl)-1H-benzimidazol-2-yl]ethyl]-guanidin; 6-[2-(4-Aminobutyl)-1H-benzimidazol-5-yl]-4,5-dihydro-5-methyl-3(2H)-pyridazinon und $N^1$-[3-(Imidazol-4-yl)propyl] -$N^2$-4-[5-(4-methyl-6-oxo-1,4,5,6-tetrahydropyridazin-3-yl)-1H-benzimidazol-2-yl]butyl]-guanidin und deren physiologisch annehmbaren Salze.

Die erfindungsgemäßen Verbindungen werden durch ein Verfahren hergestellt, das dadurch gekennzeichnet ist, daß man

a) zur Herstellung von Verbindungen der allgemeinen Formel I, bei denen $R^1$, $R^2$ und n wie oben definiert sind und A für ein Wasserstoffatom steht,

$a_1$) von einer Phthalimidverbindung der allgemeinen Formel II

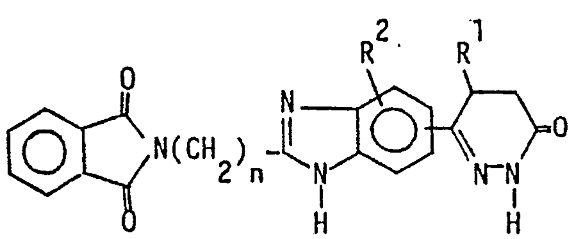 (II)

in der $R^1$, $R^2$ und n wie oben definiert sind, mit Hydrazin oder einem chemischen Äquivalent davon, einem aliphatischen primären Amin oder einer Säure die Phthalimidgruppe abspaltet und dadurch in eine Verbindung der allgemeinen Formel I überführt oder

$a_2$) eine Verbindung der allgemeinen Formel I

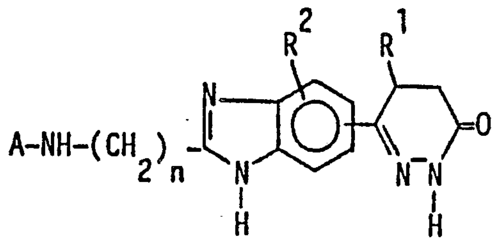

(I)

in der $R^1$, $R^2$ und n wie oben definiert sind und A für die Gruppe $-\underset{\underset{O}{\|}}{C}-R^3$ steht, wobei $R^3$ wie oben definiert ist, sauer oder basisch hydrolysiert,

b) zur Herstellung von Verbindungen der allgemeinen Formel I, bei denen $R^1$, $R^2$ und n wie oben definiert sind und A für die Gruppe $-\underset{\underset{O}{\|}}{C}-R^3$ steht,

wobei $R^3$ die oben angegebenene Definition hat, eine Verbindung der allgemeinen Formel Ia

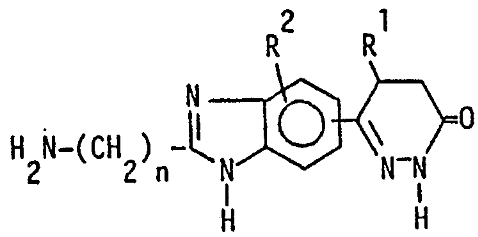

(Ia)

in der $R^1$, $R^2$ und n die oben angegebenen Bedeutungen besitzen und A für ein Wasserstoffatom steht, mit einem Acylierungsmittel der allgemeinen Formel III

$$R^3\overset{\overset{O}{\|}}{C}-Y \qquad (III)$$

in der $R^3$ die oben angegebene Bedeutung hat und Y für ein Halogenatom, insbesondere ein Chlor- oder Bromatom, den Rest $-O-\underset{\underset{O}{\|}}{C}-R^3$,

den über ein N-Atom gebundenen Rest eines Azols oder Benzazols mit mindestens 2 N-Atomen im Fünfring oder die Gruppe -OH steht, zu einer Verbindung der allgemeinen Formel I umsetzt oder

c) zur Herstellung von Verbindungen der allgemeinen Formel I, bei denen $R^1$, $R^2$ und n wie oben definiert sind und A für die Gruppe

$$-\overset{\overset{N-B}{\|}}{C}-NHR^3$$

steht, worin $R^3$ und B wie oben definiert sind,

$c_1$) eine Verbindung der allgemeinen Formel Ia

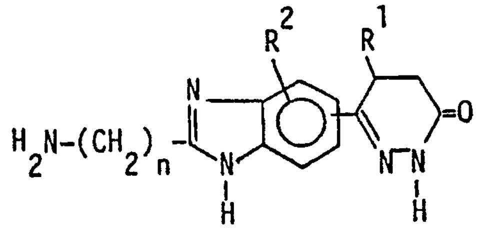 (Ia)

in der $R^1$, $R^2$ und n die oben angegebenen Bedeutungen haben, mit einer Verbindung der allgemeinen Formel IV

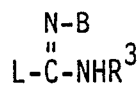 (IV)

in der B und $R^3$ wie oben definiert sind und L für eine Austrittsgruppe, zum Beispiel eine Alkylthio-, Arylthio-, Alkoxy- oder Aryloxygruppe, steht, zu einer Verbindung der allgemeinen Formel I umsetzt oder

$c_2$) eine Verbindung der allgemeinen Formel Ia

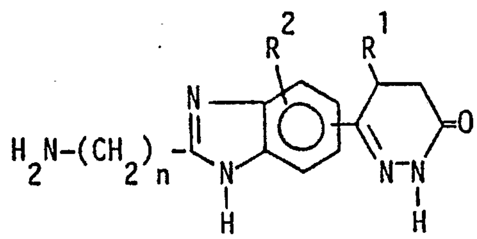 (Ia)

in der $R^1$, $R^2$ und n die oben angegebenen Bedeutungen haben, zunächst mit einer Verbindung der allgemeinen Formel V

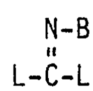 (V)

in der B und L die oben angegebenen Bedeutungen haben, zu einer Verbindung der allgemeinen Formel XIII

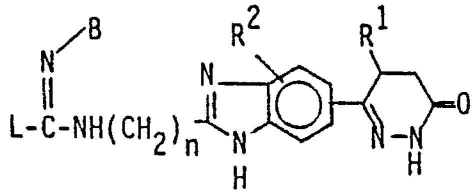 (XIII)

in der $R^1$, $R^2$, B, L und n die oben angegebenen Bedeutungen haben, umsetzt und diese sodann mit einer Verbindung der allgemeinen Formel VI

$R^3$-$NH_2$ (VI)

in der $R^3$ wie oben definiert ist, zu einer Verbindung der allgemeinen Formel I umsetzt oder

d) zur Herstellung von Verbindungen der allgemeinen Formel I, bei der $R^1$, $R^2$ und n wie oben definiert sind und A für die Gruppe

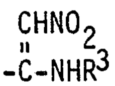

steht, in der $R^3$ die oben angegebene Bedeutung hat,

$d_1$) einer Verbindung der allgemeinen Formel Ia

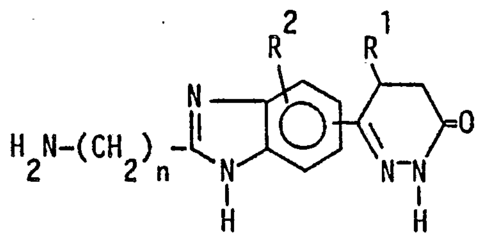
(Ia)

in der $R^1$, $R^2$ und n die oben angegebenen Bedeutungen haben, mit einer Verbindung der allgemeinen Formel VII

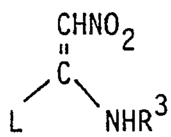
(VII)

in der L für eine Austrittsgruppe, zum Beispiel eine Alkylthio-, Arylthio-, Alkoxy- oder Aryloxygruppe, steht und $R^3$ die oben angegebene Bedeutung besitzt, zu einer Verbindung der allgemeinen Formel I umsetzt oder

$d_2$) eine Verbindung der allgemeinen Formel Ia

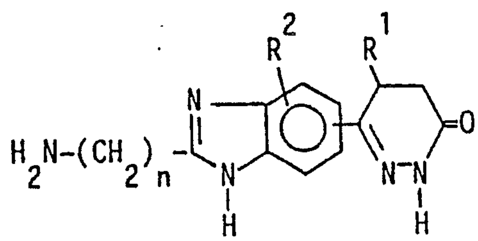
(Ia)

in der $R^1$, $R^2$ und n die oben angegebenen Bedeutungen haben, zunächst mit einer Verbindung der allgemeinen Formel VIII

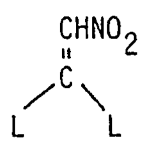
(VIII)

in der L die oben angegebene Bedeutung hat, zu einer Verbindung der allgemeinen Formel IX

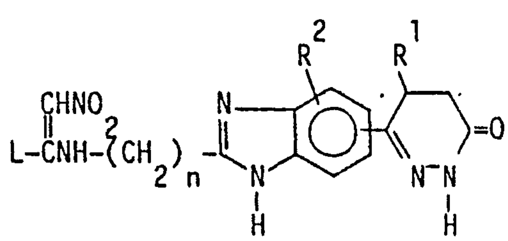
(IX)

in der $R^1$, $R^2$, n und L wie oben definiert sind, umsetzt und diese sodann mit einer Verbindung der allgemeinen Formel VI

$R^3$-$NH_2$ (VI)

in der $R^3$ wie oben definiert ist, zu einer Verbindung der allgemeinen Formel I umsetzt oder

e) zur Herstellung von Verbindungen der allgemeinen Formel I, bei denen $R^1$, $R^2$ und n wie oben definiert sind und A für die Gruppe

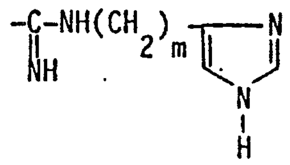

in der m den Wert 2 oder 3 hat, steht,

$e_1$) eine Verbindung der allgemeinen Formel X

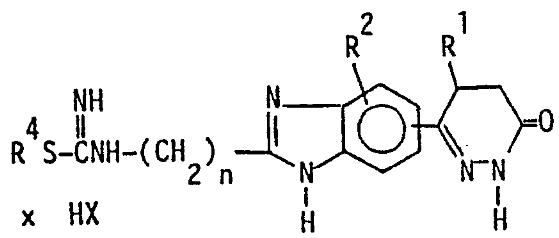

(X)

in der $R^1$, $R^2$ und n wie oben definiert sind, $R^4$ für eine gegebenenfalls substituierte $C_1$-$C_4$- Alkylgruppe oder eine Benzylgruppe steht und X für ein Halogenatom oder die Gruppe -$OSO_2OR^4$ steht, mit einem m-(Imidazol-4-yl)alkylamin der allgemeinen Formel XI

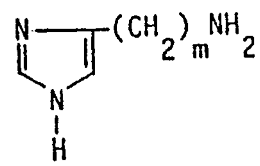

(XI)

in der m den Wert 2 oder 3 hat, zu einer Verbindung der allgemeinen Formel I umsetzt oder

$e_2$) eine Verbindung der allgemeinen Formel Ia

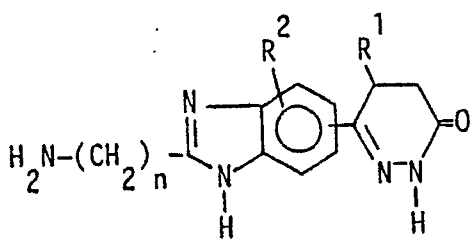

(Ia)

in der $R^1$, $R^2$ und n die oben angegebenen Bedeutungen haben, mit einer Verbindung der allgemeinen Formel XII

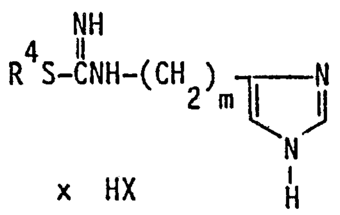

(XII)

in der m, $R^4$ und X wie oben definiert sind, zu einer Verbindung der allgemeinen Formel I umsetzt und

gegebenenfalls die nach den Verfahrensvarianten a) bis e) erhaltenen Verbindungen der allgemeinen Formel I in an sich bekannter Weise in ein physiologisch annehmbares Salz umwandelt.

Somit können die erfindungsgemäßen Verbindungen der allgemeinen Formel I nach mehreren verschiedenen Verfahrensvarianten hergestellt werden:

1.) Verbindungen der allgemeinen Formel I, bei denen $R^1$, $R^2$ und n die oben angegebenenen Bedeutungen haben und A für ein Wasserstoffatom steht, werden erhalten

1a.) durch Umsetzen einer Verbindung der allgemeinen Formel II

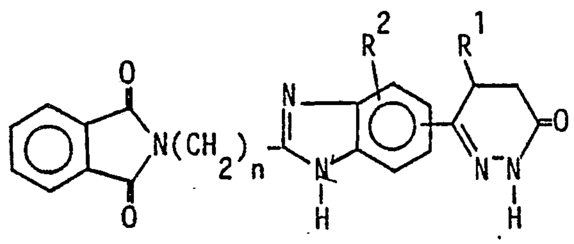

(II)

in der $R^1$, $R^2$ und n wie oben definiert sind, mit Hydrazin bzw. einem chemischen Äquivalent davon, einem primären, aliphatischen Amin oder einer Säure. Die Verbindungen der allgemeinen Formel II können beispielsweise durch die in der DE-OS 28 37 161 beschriebenen Umsetzungen oder durch analoge Umsetzungen hergestellt werden. Mit "chemischen Äquivalenten des Hydrazins" werden dabei Hydrazinhydrat, Hydrazinethanolat bzw. ähnliche Solvate oder seine Salze bezeichnet. Die Umsetzungen mit Hydrazin oder einem Amin, wie zum Beispiel Methylamin oder Ethanolamin, erfolgen vorzugsweise mit einem Überschuß an Reagenz und in einem polaren Lösungsmittel, wie zum Beispiel einem Alkohol, wie Methanol, Ethanol oder Isopropanol. Die Reaktionen werden bei Temperaturen von Raumtemperatur bis zum Siedepunkt des verwendeten Lösungsmittels, vorzugsweise bei Rückflußtemperatur, und bei der Verwendung von Aminen eventuell unter erhöhtem Druck durchgeführt. Die saure Hydrolyse der Verbindungen der allgemeinen Formel II wird in wäßrigen Mineralsäuren, wie Chlor-, Brom-oder Jodwasserstoffsäure, Schwefelsäure oder Phosphorsäure, in verdünnten organischen Säuren, wie zum Beispiel Essigsäure, bzw. in Gemischen von wäßrigen Mineralsäuren und organischen Säuren bei erhöhter Temperatur, vorzugsweise bei Rückflußtemperatur, durchgeführt. Bei dieser Umsetzung wird von der Verbindung der allgemeinen Formel II die Phthalimidgruppe abgespalten, wodurch die Verbindung der allgemeinen Formel I erhalten wird.

Die weitere Möglichkeit ist

1b.) die saure oder basische Hydrolyse einer Verbindung der allgemeinen Formel I, in der $R^1$, $R^2$ und n wie oben definiert sind und A für die Gruppe $-\underset{\underset{O}{\|}}{C}-R^3$ steht,

wobei $R^3$ wie oben definiert ist. Die saure Hydrolyse wird in wäßrigen Mineralsäuren, wie Chlor- oder Bromwasserstoffsäure oder Schwefelsäure, vorzugsweise Chlorwasserstoffsäure, und bei erhöhten Temperaturen, vorzugsweise Rückflußtemperatur, durchgeführt. Die basische Hydrolyse erfolgt in verdünnten Lösungen von Alkali- oder Erdalkalicarbonaten bzw. Alkali- oder Erdalkalihydroxiden in Wasser, niedrigen Alkoholen oder Gemischen von beiden und bei Temperaturen von Raumtemperatur bis zur Rückflußtemperatur des verwendeten Lösungsmittels.

2.) Verbindungen der allgemeinen Formel I, bei denen $R^1$, $R^2$ und n wie oben definiert sind und A für die Gruppe $-\underset{\underset{O}{\|}}{C}-R^3$ steht,

worin $R^3$ wie oben definiert ist, werden erhalten durch Umsetzung einer Verbindung der allgemeinen Formel Ia, in der $R^1$, $R^2$ und n die oben angegebenen Bedeutungen haben und A für ein Wasserstoffatom steht, mit einem Acylierungsmittel der allgemeinen Formel III

$$R^3-\overset{\overset{O}{\|}}{C}-Y \qquad (III)$$

in der $R^3$ die oben angegebene Bedeutung hat und Y für ein Halogenatom, insbesondere ein Chlor- oder Bromatom, den Rest $-O-\underset{\underset{O}{\|}}{C}-R^3$,

wobei $R^3$ die oben angegebene Bedeutung hat, den über ein Stickstoffatom gebundenen Rest eines Azols oder Benzazols mit mindestens 2 Stickstoffatomen im Fünfring oder die Gruppe -OH steht. Beispiele für die genannten Azole bzw. Benzazole sind der Imidazol-, 1,2,4-Triazol-, Tetrazol-, Benzimidazol- oder Benzotriazolring. Wird als Acylierungsmittel eine Verbindung der allgemeinen Formel III verwendet, bei der Y für die Gruppe -OH steht, so ist der Zusatz eines Aktivierungsmit tels zweckmäßig, das die Aufgabe hat, das Acylierungspotential der Carbonsäure zu erhöhen. Geeignete derartige Aktivierungsmittel sind wasserentziehende bzw. wasserbindende Mittel, wie zum Beispiel Carbodiimide, oder solche Agenzien, welche die Carbonsäuren in die entsprechenden, als Acylierungsmittel wirkenden Säurehalogenide, Anhydride, gemischte Carbonsäure-Kohlensäure-Anhydride oder Azolide überführen, wie zum Beispiel Phosgen, Chlorameisensäureester oder N,N′-Carbonyldiimidazol. Die Umsetzung zwischen dem Acylierungsmittel der allgemeinen Formel III und der Verbindung der allgemeinen Formel I, in der $R^1$, $R^2$ und n die oben angegebenen Bedeutungen haben und A für ein Wasserstoffatom steht, wird zweckmäßigerweise in einem inerten Lösungsmittel, zum Beispiel einem halogenierten Kohlenwasserstoff, Ether, Pyridin, Dimethylformamid, oder einem aromatischen Kohlenwasserstoff, bei Temperaturen zwischen -20 °C und dem Siedepunkt des verwendeten Lösungsmittels durchgeführt. Das Molverhältnis zwischen dem Acylierungsmittel der Formel III und der Verbindung der allgemeinen Formel I beträgt normalerweise 3:1 bis 1:1, vorzugsweise 2:1 bis 1:1. Falls bei der Acylierungsreaktion eine Säure abgespalten wird, ist der Zusatz eines Säurefängers, wie zum Beispiel eines tertiären Amins, wie Triethylamin oder Pyridin, zweckmäßig.

3.) Verbindungen der allgemeinen Formel I, in denen $R^1$, $R^2$ und n wie oben definiert sind und A für die Gruppe

$$-\overset{\overset{N-B}{\|}}{C}-NHR^3$$

steht, worin $R^3$ und B wie oben definiert sind, können nach zwei verschiedenen Verfahrensvarianten erhalten werden:

Nach einer Variante (3a.) wird eine Verbindung der allgemeinen Formel Ia, in der $R^1$, $R^2$ und n die oben angegebenen Bedeutungen haben, mit einer Verbindung der allgemeinen Formel IV

$$L-\overset{\overset{N-B}{\|}}{C}-NHR^3 \qquad (IV)$$

in der B und $R^3$ wie oben definiert sind und L für eine Austrittsgruppe, wie zum Beispiel eine Alkylthio-, Arylthio-, Alkoxy- oder Aryloxygruppe, steht, umgesetzt. Bevorzugte Austrittsgruppen sind die Phenoxy- und die Methylthiogruppe. Die Umsetzung wird vorzugsweise mit äquimolaren Mengen der Reaktionspartner unter Verwendung eines Lösungsmittels und bei erhöhter Temperatur, vorzugsweise Rückflußtemperatur des verwendeten Lösungsmittels, durchgeführt.

Nach der zweiten Verfahrensvariante wird (3b.) in einem zweistufigen Prozeß zunächst die Verbindung der allgemeinen Formel Ia, in der $R^1$, $R^2$ und n die oben angegebenen Bedeutungen haben, mit einer Verbindung der allgemeinen Formel V

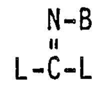 (V)

in der B und L die oben genannten Bedeutungen haben, umgesetzt. Diese Stufe führt zu einer Zwischenverbindung der oben angegebenen Formel XIII, in der $R^1$, $R^2$, B, L und n die oben genannten Bedeutungen besitzen. Diese Verbindung wird sodann mit einer Verbindung der allgemeinen Formel VI

$R^3$-$NH_2$ (VI)

in der $R^3$ wie oben definiert ist, weiter umgesetzt.
In der ersten Stufe dieser Verfahrensvariante werden die Reaktionspartner in äquimolaren Mengen in einem inerten, aprotischen Lösungsmittel, zum Beispiel einem Ether, wie Tetrahydrofuran, oder Lösungsmitteln, wie Acetonitril, Dimethylformamid oder Dimethylsulfoxid, umgesetzt. Die Umsetzung kann prinzipiell bei Temperaturen von -20 °C bis zur Rückflußtemperatur des verwendeten Lösungsmittels erfolgen, bevorzugt werden Temperaturen von 10 bis 30 °C.
Bezüglich der Reaktionsbedingungen der zweiten Stufe gilt hinsichtlich Lösungsmittel und Reaktionstemperatur das im Zusammen hang mit der Verfahrensvariante 3a Gesagte. Das Amin der allgemeinen Formel VI wird bevorzugt im großen Überschuß, vorzugsweise in 5- bis 10fachem Überschuß, eingesetzt.
Die intermediär auftretende Zwischenverbindung der allgemeinen Formel XIII

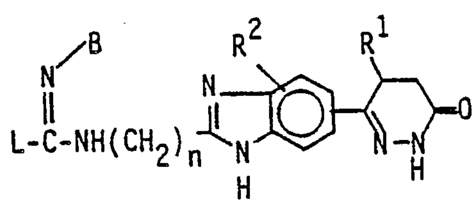 (XIII)

in der $R^1$, $R^2$, B, L und n wie oben definiert sind, kann mit herkömmlichen Arbeitsweisen isoliert werden, vorzugsweise wird sie jedoch ohne Isolierung und Reinigung mit dem Amin der Formel VI direkt weiter umgesetzt.

4.) Verbindungen der allgemeinen Formel I, in denen $R^1$, $R^2$ und n wie oben definiert sind und A für die Gruppe

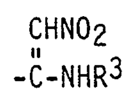

steht, wobei $R^3$ die oben genannte Bedeutung hat, können nach zwei verschiedenen Verfahrensvarianten erhalten werden:

Nach der einen Variante (4a.) wird eine Verbindung der allgemeinen Formel Ia, in der $R^1$, $R^2$ und n die oben angegebenen Bedeutungen haben, mit einer Verbindung der allgemeinen Formel VII

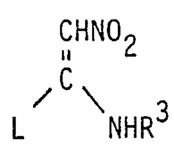 (VII)

in der L und $R^3$ wie oben definiert sind, zu einer Verbindung der allgemeinen Formel I umgesetzt.
Gemäß der weiteren Verfahrensvariante (4b.) wird eine Verbindung der allgemeinen Formel Ia, in der $R^1$, $R^2$ und n die oben an gegebenen Bedeutungen haben, zunächst mit einer Verbindung der allgemeinen Formel VIII

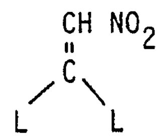

(VIII)

in der L die oben angegebene Bedeutung besitzt, zu einer Zwischenverbindung der oben angegebenen allgemeinen Formel IX, in der $R^1$, $R^2$, L und n wie oben definiert sind, umgesetzt. Diese wird sodann mit einer Verbindung der allgemeinen Formel VI

$R^3$-$NH_2$ (VI)

in der $R^3$ wie oben definiert ist, zu einer Verbindung der allgemeinen Formel I umgesetzt.

Als Austrittsgruppe L in den Verbindungen der Formeln VII und VIII wird in beiden Verfahrensvarianten die Alkylthiogruppe, insbesondere die Methylthiogruppe, bevorzugt. Die Verbindungen der Formeln VII und VIII werden vorzugsweise in äquimolaren Mengen, bezogen auf die Verbindungen der Formel Ia, eingesetzt. Die Umsetzungen werden in polaren Lösungsmitteln, wie Acetonitril, Pyridin, Dimethylformamid, oder Alkoholen, vorzugsweise sekundären oder tertiären Alkoholen, wie zum Beispiel Isopropanol, und bei erhöhter Temperatur, insbesondere bei Rückflußtemperatur, durchgeführt. In der zweiten Stufe des zweistufigen Verfahrens wird die Zwischenverbindung der allgemeinen Formel IX mit einem großen Überschuß des Amins der Formel VI, vorzugsweise 10-bis 30fachen Überschuß, in einem Lösungsmittel, dessen Auswahl für den Erfolg der Reaktion unkritisch ist, bei erhöhter Temperatur, vorzugsweise Rückflußtemperatur des verwendeten Lösungsmittels, umgesetzt.

5.) Verbindungen der allgemeinen Formel I, in denen $R^1$, $R^2$ und n wie oben definiert sind und A für die Gruppierung

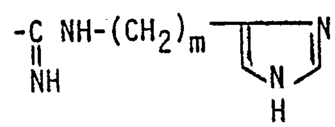

in der m den Wert 2 oder 3 hat, steht, werden erhalten

5a.) durch Umsetzung einer Verbindung der allgemeinen Formel X

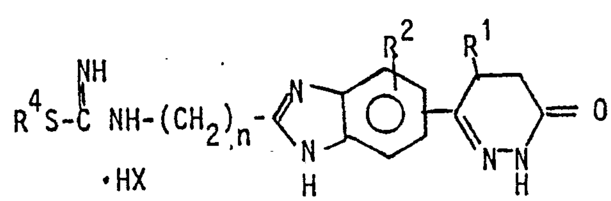

(X)

in der $R^1$, $R^2$ und n wie oben definiert sind, $R^4$ für eine gegebenenfalls substituierte $C_1$-$C_4$-Alkylgruppe (wobei die Alkylgruppe beispielsweise ein- oder zweifach mit einem Halogenatom, zum Beispiel einem Chlor-, Brom- oder Jodatom, einer $C_1$-$C_4$-Alkoxygruppe, zum Beispiel einer Methoxy- oder Ethoxygruppe, und/oder einem Arylrest, insbesondere einem Phenylrest, substituiert ist) oder eine Benzylgruppe steht und X ein Halogenatom oder die Gruppe -$OSO_2OR^4$ (worin $R^4$ die oben angegebene Bedeutung hat) bedeutet, mit einem m-(Imidazol-4-yl)alkylamin der allgemeinen Formel XI

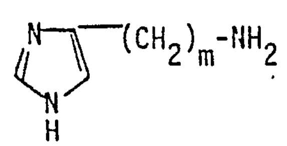

(XI)

in der m den Wert 2 oder 3 hat,oder

5b.) durch Umsetzung einer Verbindung der allgemeinen Formel Ia, in der $R^1$, $R^2$ und n wie oben definiert sind, mit einer Verbindung der allgemeinen Formel XII

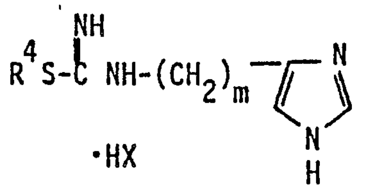

(XII)

in der $R^4$, X und m die oben genannten Bedeutungen besitzen.
Die Reaktionspartner werden vorzugsweise in äquimolaren Mengen in einem polaren, aprotischen Lösungsmittel, wie Pyridin, Dimethylformamid, Dimethylsulfoxid oder Acetonitril, und bei erhöhter Temperatur, insbesondere bei Rückflußtemperatur des verwendeten Lösungsmittels, miteinander umgesetzt.

Die nach den einzelnen Verfahrensvarianten erhaltenen Verbindungen werden in üblicher Weise isoliert und gereinigt, beispielsweise durch Umkristallisation, chromatographische Arbeitsweisen etc.

Die bei den einzelnen Verfahrensvarianten erhaltenen Verbindungen können gegebenenfalls in ihr physiologisch annehmbares Salz umgewandelt werden. Diese Salze können zum Beispiel mit Mineralsäuren, wie Chlor-, Brom-, Jodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure, Embonsäure etc., gebildet werden.

Die erfindungsgemäßen Verbindungen der allgemeinen Formel I können sowohl in einer Reihe von tautomeren Formen als auch in mehreren stereoisomeren Formen vorliegen. Die Erfindung umfaßt daher neben den Salzen und Hydraten der oben beschriebenen Verbindungen der allgemeinen Formel I auch alle tautomeren und stereoisomeren Formen.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung eines oder mehrerer pharmazeutisch annehmbarer Träger oder Verdünnungsmittel hergestellt werden.

Die erfindungsgemäßen Verbindungen können daher für die orale, bukkale, topische, parenterale oder rektale Verabreichung formuliert werden.

Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind.

Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/oder Dispergierungsmittel, enthalten.

Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, je nach Zustand des Patienten. Im Einzelfall kann es gegebenenfalls erfor derlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, in denen mit weniger als der oben genannten Mindestmenge ausgekommen werden kann, während in anderen Fällen die genannte

obere Grenze überschritten werden muß.

Die erfindungsgemäßen Benzimidazole der allgemeinen Formel I zeigen bei guter oraler Verfügbarkeit interessante pharmakologische Eigenschaften, im speziellen cardiovaskuläre, insbesondere positiv inotrope und blutdrucksenkende Wirkungen. So zeigen sie eine ausgezeichnete cardiotone Wirksamkeit in einer Reihe von pharmakologischen Standardmodellen, zum Beispiel in vitro im isolierten, perfundierten Langendorff-Herzen oder in vivo am narkotisierten Meerschweinchen, wo sie eine deutliche Steigerung der Kontraktilität bewirken.

1. Untersuchungen am isolierten, perfundierten Langendorff-Herzen (Meerschweinchen)

a) Methode

Zur Bestimmung der hämodynamischen Effekte der erfindungsgemäßen Verbindungen an isolierten, perfundierten Meerschweinchenherzen wurde die Anordnung von Langendorff nach P.R. Beckett (J. Pharm. Pharmacol. 22, 818 (1970)) und R.M. Abel und R.L. Reis (Circ. Res. 27, 961 (1970)) modifiziert. Die spontan schlagenden Meerschweinchenherzen waren linksventrikulär katheterisiert und wurden mit Lösungen der Prüfsubstanzen in physiologischer Kochsalzlösung/Ethanol (9:1) in Konzentrationen von $10^{-4}$ bis $10^{-8}$ mol/l mit einem konstanten Perfusionsdruck von 60 mm Hg perfundiert.

b) Meßwerte

| Beispiel Nr. | Konzentrationsbereich (mol/l) | Maximale prozentuale Veränderungen gegenüber den Kontrollen | | |
|---|---|---|---|---|
| | | Kontraktilität dp / dt | Koronarfluß | Herzfrequenz |
| 1 | $10^{-8}$-$10^{-4}$ | + 76% | + 25% | + 11% |
| 6 | $10^{-7}$-$10^{-5}$ | + 161% | + 52% | + 20% |
| Pimobendan (Vergleich) | $10^{-7}$-$10^{-5}$ | + 99% | + 76% | + 8% |

2. Hämodynamische Charakterisierung am narkotisierten Meerschweinchen In-vivo-Modell

a) Methode

Die Tiere werden mit Urethan (1,5 g/kg) narkotisiert. Zur volumenkontrollierten Beatmung wird die Trachea kanüliert. Danach erfolgt die operative Freilegung beider Karotiden; über die rechte Karotis wird ein Tip-Katheter (3F) eingeführt, der unter fortlaufender Druckregistrierung dann über die Aorta ascendens in den linken Ventrikel vorgeführt wird. Die erfolgreiche Passage der Aortenklappen wird hierbei durch die typische linksventrikuläre Druckkurve erkannt. Über die linke Karotis wird zur Thermodilution ein Thermistorfühler (3F, F. Edwards) in den Aortenbogen vorgeschoben. Der Thermistorfühler hat gleichzeitig ein Lumen zur arteriellen Blutdruckregistrierung. Zur Applikation des Kälteinjektats (0,2 ml 0,9% NaCl, 15 °C) wird durch die rechte Vena jugularis ein Katheter vor den rechten Vorhof plaziert. Die Registrierung des EKGs erfolgt in der 1. Ableitung. Alle Substanzen sind in physiologischer Kochsalzlösung gelöst und werden über die linke Vena jugularis infun diert (Infusionsvolumen 0,02 ml/min); die Applikation erfolgt nach hämodynamischer Stabilierung und unter β-Blockade (Metoprolol 2 mg/kg i.m.). Alle Kreislaufparameter werden kontinuierlich auf einem Direktschreiber registriert. Die Berechnung der Pulsfrequenz erfolgt aus dem EKG, die Kontraktilität (dp/dt) wird über die Volumenkurve berechnet, das Herzzeitvolumen aus der Thermodilutionskurve.

b) Meßwerte

| Beispiel Nr. | Dosis (µg/kg/min) | Maximale prozentuale Veränderungen gegenüber den Ausgangswerten | | |
|---|---|---|---|---|
| | | Kontraktilität dp / dt | Blutdruck sys. | Herzfrequenz |
| 1 | 10 | + 38% | - 25% | + 20% |
| 6 | 10 | + 97% | - 15% | + 17% |
| Pimobendan (Vergleich) | 10 | + 10% | - 51% | + 3% |

Die Phosphodiesterase-Hemmwirkung der erfindungsgemäßen Verbindungen wurde an Phosphodiesterase-Typ III aus Rinderherz (Sigma Chemie, BRD) nach den Methoden von W. Diederen und H. Weisenberger (Arzneim.-Forschung 31, 177 (1981)) (Methode A) bzw. von M.A. Appleman und W.L. Terasaki (Advances in Cyclic Nucelotide Research, Bd. 5, Raven Press, New York (1975), S. 153) (Methode B) bestimmt. Die Tabelle gibt die prozentuale Hemmung der PDE-III-Aktivität wieder.

| | | Konzentration (mol/l) | | | |
|---|---|---|---|---|---|
| | | $10^{-7}$ | $10^{-6}$ | $10^{-5}$ | $10^{-4}$ |
| Beispiel 1 | Methode A | 0 | 0 | 7 | 10 |
| | Methode B | 0 | 0 | 0 | 4 |
| Pimobendan (Vergleich) | Methode A | 7 | 15 | 30 | - |
| | Methode B | - | 5 | 19 | 57 |

Beispiel 1

6-[2-(3-Aminopropyl)-1H-benzimidazol-5-yl]-4,5-dihydro-5-methyl-3(2H)-pyridazinon

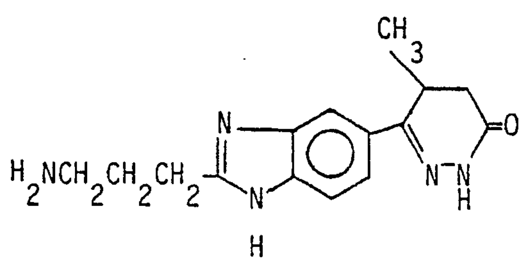

a) 6-[3-Nitro-4-[[4-(phthalimido)butyryl]amino]phenyl]-4,5-dihydro-5-methyl-3(2H)-pyridazinon

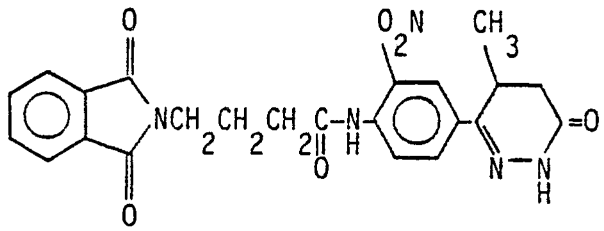

49.7 g (200 mmol) 6-(4-Amino-3-nitro-phenyl)-4,5-dihydro-5-methyl-3(2H)pyridazinon und 52,9 g (210 mmol) 4-Phthalimido-buttersäurechlorid werden in 500 ml Pyridin 4 h bei 100° C Innentemperatur gerührt. Das abgekühlte Reaktionsgemisch wird auf 1500 ml Eiswasser gegeben und das sich abscheidende Öl mit 2 x 1000 ml Dichlormethan extrahiert. Die vereinigten organischen Phasen werden mit 250 ml Wasser gewaschen, mit Natriumsulfat getrocknet und filtriert. Der nach Abdampfen des Lösungsmittels i. Vak. erhaltene Rückstand wird aus Ethylacetat umkristallisiert. Man erhält 69,6 g (75 %) gelbe Kristalle.
$C_{23}H_{21}N_5O_5$ (463,45)
Schmp. 182 - 184° C

b) 6-[3-Amino-4-[[4-(phthalimido)butyryl] amino]phenyl]-4,5-dihydro-5-methyl-3 (2H)-pyridazinon

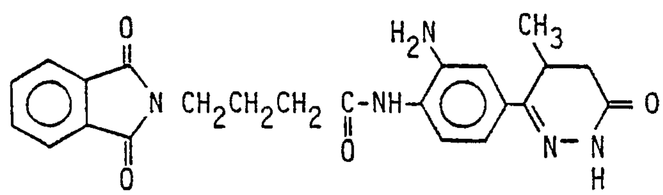

Eine Suspension von 15,8 g (34 mmol) 6-[3-Nitro-4-[[4-(phthalimido) butyryl]amino]phenyl]-4,5-dihydro-5-methyl-3(2H)-pyridazinon in 350 ml Ethanol wird bei 50° C und einem Wasserstoffdruck von 5 bar in Gegenwart von 1,6 g Pd-Kohle (10 % Pd) hydriert. Nach beendeter Wasserstoffaufnahme (ca. 8 h) wird die Lösung mit 350 ml Dichlormethan verdünnt und über eine Supercel-Schicht filtriert. Das Filtrat ergibt nach Eindampfen i.vak. 14.1 g (96 %) eines braungelben Feststoffs, der ohne Reinigung in die nächste Stufe eingesetzt wird.
$C_{23}H_{23}N_5O_4$ (433,47)
Schmp. 190 - 191° C (aus Ethanol/Acetonitril 4:1)

c) 6-[2-(3-Phthalimidopropyl)-1H- benzimidazol-5-yl-4,5-dihydro-5-methyl-3(2H)]-pyridazinon

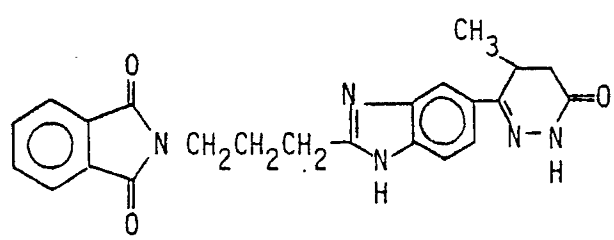

14,1 g (32,5 mmol) 6-[3-Amino-4-[[4-(phthalimido)butyryl]amino]phenyl]-4,5-dihydro-5-methyl-3(2H)-pyridazinon werden in 100 ml Eisessig 2 h unter Rückfluß gekocht. Die Lösung wird i. Vak. weitgehend eingedampft und der Rückstand mit 200 ml Ethanol versetzt. Nach 30-minütigem Rühren bei Raumtemperatur wird auf 5° C abgekühlt und der entstandene Niederschlag abgesaugt. Man erhält 7.7 g (57 %) eines Feststoffs mit dem Schmp. 260 -261° C.

$C_{23}H_{21}N_5O_3$ (415,45)

d) 6-[2-(3-Aminopropyl)-1H-benzimidazol-5-yl]-4,5-dihydro-5-methyl-3(2H)-pyridazinon

25,9 g (62 mmol) 6-[2-(3-Phthalimidopropyl)-1H-benzimidazol-5-yl]-4,5-dihydro- 5-methyl-3(2H)-pyridazinon und 16.0 ml (330 mmol) Hydrazinhydrat werden in 300 ml Ethanol 1,5 h unter Rückfluß gekocht. Die erhaltene, dicke Suspension wird i.Vak. eingedampft, der Rückstand in 500 ml 2N Salzsäure aufgenommen und die Lösung filtriert. Das Filtrat wird i. Vak. eingedampft, mit 100 ml Ethanol versetzt und abermals eingedampft. Der verbleibende Feststoff wird in 200 ml gesättigter Kaliumcarbonat-Lösung aufgenommen und die Mischung mit 3 x 100 ml Isopropanol extrahiert. Die vereinigten organischen Phasen werden getrocknet und i.Vak. eingedampft. Nach Umkristallisation aus Ethanol/Wasser erhält man 13,9 g (79 %) eines farblosen Feststoffs.
$C_{15}H_{19}N_5O$ (285,35)
Schmp. 228,5 - 229,5° C
Monohydrochlorid Schmp. 209 - 211° C
Dihydrochlorid Schmp. 213 - 217° C

Beispiel 2

6-[2-(3-Acetamidopropyl)-1H-benzimidazol-5-yl]-4,5-dihydro-5-methyl-3(2H)-pyridazinon

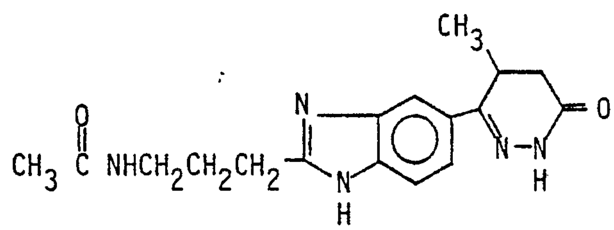

0,50 g (1,75 mmol) 6-[2-(3-Aminopropyl)-1H-benzimidazol-5-yl]-4,5-dihydro-5-methyl-3(2H)-pyridazinon werden in 20 ml Eisessig mit 0,34 ml (3,50 mmol) Acetanhydrid 45 Minuten gekocht. Die abgekühlte Lösung wird i.Vak. weitgehend eingeengt, der Rückstand in 10 ml Wasser aufgenommen und die Lösung mit 10-%igem Ammoniak auf pH 9 gestellt. Nach Einengen der Lösung auf ca. 1/3 des Volumens und Kühlen im Eisbad kristallisiert ein gelblicher Feststoff, der abgesaugt und dann in 30 ml Acetonitril aufgekocht wird. Der verbleibende Feststoff wird abgesaugt, mit 10 ml Acetonitril gewaschen und i.Vak. getrocknet. Man erhält 0,51 g (89 %) farblose Kristalle vom Schmp. 244-246 °C.
$C_{17}H_{21}N_5O_2$ (327,39)
$^1$H-NMR-Daten (DMSO-$d_6$, TMS als interner Standard)
$\delta$ = 1,1 (d) 3H
1,85 (s) 3H
1,7-3,0 (m) 6H
3,1-3,4 (m) 2H
3,55 (t) 1H
7,5-8,2 (m) 4H, 1H austauschbar mit $D_2O$
11,0 (s) 1H austauschbar mit $D_2O$
12,5 (breit) 1H austauschbar mit $D_2O$ ppm

Beispiel 3

$N^1$-Cyano-$N^2$-methyl-$N^3$-[3-[5-(4-methyl-6-oxo-1,4,5,6-tetrahydropyridazin-3-yl)-1H-benzimidazol-2-yl] propyl] guanidin

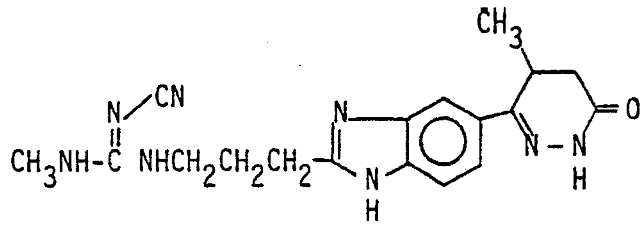

0.50 g (1.75 mmol) 6-[2-(3-Aminopropyl)-1H-benzimidazol-5-yl]-4,5-dihydro-5-methyl-3(2H)- pyridazinon und 0,31 g (1.76 mmol) N[1]-Cyano-N[2]-methyl-O-phenyl-isoharnstoff werden in 20 ml Isopropanol 3h unter Rückfluß gekocht. Das Reaktionsgemisch wird noch heiß filtriert und das Filtrat i.Vak. eingedampft. Der blaßgelbe Rückstand wird an Kieselgel mit Dichlormethan/Methanol (9:1) chromatographiert und ergibt nach Eindampfen der Hauptfraktion i.Vak. 0,42 g (65 %) eines farblosen, amorphen Feststoffs.

$C_{18}H_{22}N_8O$ (366,43)

$^1$H-NMR-Daten ($CD_3OD$, TMS als interner Standard)

$\delta$ = 1,2 (d) 3H

1,9-3,1 (m) 6H

2,85 (s) 3H

3,3-3,7 (m) 3H

4,9 (breit) 4H austauschbar mit $D_2O$

7,6-8,0 (m) 2H

8,15 (s) 1H ppm

Beispiel 4

N[1]-Cyano-N[2]-isopropyl-N[3]-[3-[5-(4-methyl 6-oxo-1,4,5,6-tetrahydropyridazin-3-yl)-1H-benzimidazol-2-yl] propyl] guanidin

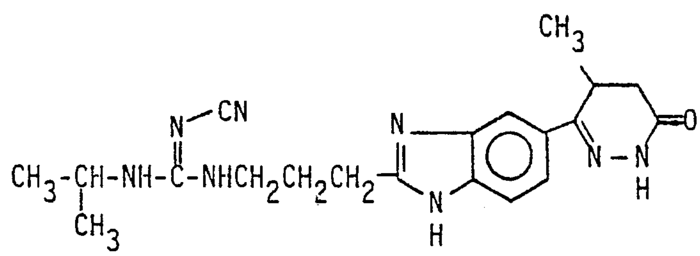

Analog zu Beispiel 3 erhält man aus 0,70 g (2,45 mmol)6-[2-(3-Aminopropyl)-1H-benzimidazol-5-yl]-4,5-dihydro-5- methyl-3(2H)-pyridazinon und 0,50 g (2,46 mmol) N[1]-Cyano-N[2]-isopropyl- O-phenyl-isoharnstoff 0,43 g (44 %) eines farblosen, amorphen Feststoffs.

$C_{20}H_{26}N_8O$ (394,48)

$^1$H-NMR-Daten (DMSO-$d_6$, TMS als interner Standard)

$\delta$ = 1,0-1,3 (2d) 9H

1,8-3,1 (m) 6H

3,2-3,7 (m) 3H

3,8-4,2 (m) 1H

6,9 (d) 1H austauschbar mit $D_2O$

7,3 (t) 1H austauschbar mit $D_2O$

7,6-8,2 (m) 3H

11,1 (s) 1H austauschbar mit $D_2O$

12.6 (breit) 1H austauschbar mit $D_2O$ ppm

Beispiel 5

1-Methylamino-1-[3-[5-(4-methyl-6-oxo-1,4,5,6-tetrahydropyridazin-3-yl)-1H-benzimidazol-2-yl] propylamino]-2-nitroethen

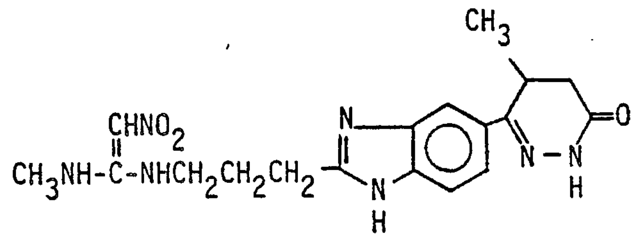

2,00 g (7,0 mmol) 6-[2-(3-Aminopropyl)-1H-benzimidazol-5-yl]-4,5-dihydro-5-methyl-3(2H)-pyridazinon und 1,16 g (7,0 mmol) 1,1-Bis-(methylthio)-2-nitroethen werden in 50 ml Isopropanol 1h unter Rückfluß gekocht. Nach Abkühlen wird die Lösung i.Vak. eingedampft und die Zwischenstufe ohne Reinigung mit 10 ml einer Lösung von Methylamin in Methanol (11 Mol/l) versetzt. Die Lösung wird 30 Minuten unter Rückfluß gekocht und dann i.Vak. eingeengt. Der Rückstand wird aus Methanol umkristallisiert. Man erhält 0,72 g (27 %) eines gelben Feststoffs vom Schmp. 168-170 °C.

$C_{18}H_{23}N_7O_3$ (385,43)

$^1$H-NMR-Daten (DMSO-$d_6$, TMS als interner Standard)

$\delta$ = 1,1 (d) 3H

1,9-3,1 (m) 9H

3,2-3,7 (m) 3H

6,7 (s) 1H

7,6-8,2 (m) 3H

10,3 (breit) 2H austauschbar mit $D_2O$

11,1 (s) 1H austauschbar mit $D_2O$

12,6 (breit) 1H austauschbar mit $D_2O$ ppm

Beispiel 6

N[1]- [3-(Imidazol 4-yl)propyl]-N[2]-[3-[ 5-(4-methyl-6-oxo-1,4,5,6-tetrahydropyridazin-3 yl)-1H-benzimidazol 2 yl]propyl]guanidin

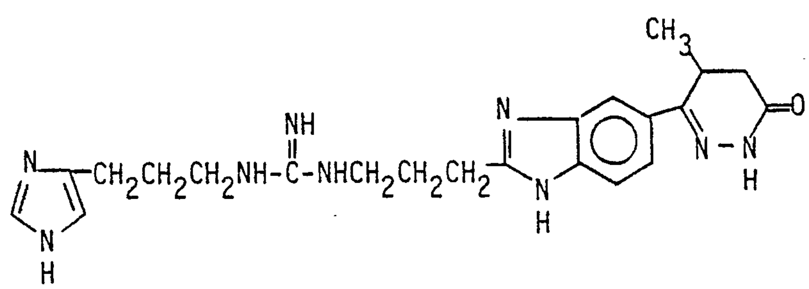

0,77 g (1,58 mmol) N-[3-[5-(4-Methyl-6-oxo-1,4,5,6-tetrahydropyridazin-3-yl)-1H-benzimidazol-2-yl] propyl] -S-methyl-isothiuroniumjodid und 0,20 g (1.59 mmol) 3-(Imidazol-4-yl)propylamin werden in 30 ml Pyridin 3,5 h unter Rückfluß gekocht. Die abgekühlte Lösung wird i.Vak. eingedampft und der Rückstand an Kieselgel mit Ethylacetat/Methanol/konz. Ammoniak gesättigt mit Ammoniumchlorid (50:47,5:2,5) als Laufmitel chromatographiert. Die Hauptfraktion (Rf-Wert 0,39) wird i.Vak. eingedampft, der Rückstand mit 10 ml gesättigter Kaliumcarbonat-Lösung aufgenommen und die wässrige Phase dreimal mit 20 ml Isopropanol extrahiert. Die organische Phase wird getrocknet, filtriert und i.Vak. eingedampft. Es verbleiben 0.32 g (47 %) eines farblosen amorphen Feststoffs.

$C_{22}H_{29}N_9O$ (435,53)

$^1$H-NMR-Daten ($CO_3OD$, TMS als interner Standard)
$\delta$ = 1,2 (d) 3H
1,7-3,7 (m) 15H
5,2 (breit) 6H austauschbar mit $D_2O$
6.95 (s) 1H
7,4-8,2 (m) 4H ppm

Beispiel 7

$N^1$-Cyano-$N^2$-[3-[5-(4-methyl-6 oxo-1,4,5,6-tetrahydropyridazin-3-yl)-1H-benzimidazol-2 yl]propyl]guanidin

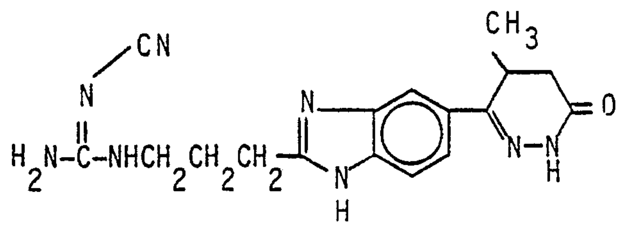

Analog zu Beispiel 3 erhält man aus 0,50 g (1,75 mmol) 6-[2-(3-Aminopropyl)-1H-benzimidazol-5-yl]-4,5 dihydro-5-methyl-3(2H)-pyridazinon und 0,30 g (1,86 mmol) N-Cyano-O-phenyl-isoharnstoff nach chromatographischer Reinigung an Kieselgel mit Ethylacetat/Ethanol/Ammoniak (70:30:2) als Eluent 0,31 g (50 %) eines gelben, amorphen Feststoffs.

$C_{17}H_{20}N_8O$ (352,40)
$^1$H-NMR-Daten ($CD_3OD$, TMS als interner Standard)
$\delta$ = 1,2 (d) 3 H
1,9 - 2,3 (m) 2 H
2,5 - 3,7 (m) 7 H
4,9 (breit) 5 H, austauschbar mit $D_2O$
7,6 - 8,2 (m) 3 H ppm.

Beispiel 8

$N^1$-Benzyl-$N^2$-cyano-$N^3$-[3-[5-(4-methyl-6-oxo-1,4,5,6-tetrahydropyridazin-3-yl)-1H-benzimidazol-2-yl]propyl]-guanidin

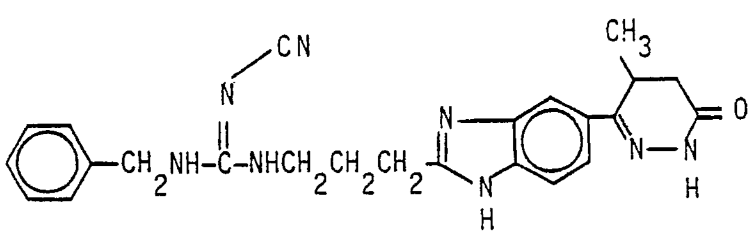

0,44 g (1,54 mmol) 6-[2-(3-Aminopropyl)-1H- benzimidazol-5-yl]-4,5-dihydro-5-methyl-3(2H)-pyridazinon und 0,43 g (1,71 mmol) $N^1$-Benzyl-$N^2$-cyano- O-phenyl-isoharnstoff werden in 20 ml Isopropanol 4 h unter Rückfluß gekocht. Der nach Abkühlen der Lösung auf Raumtemperatur erhaltene Feststoff wird abgesaugt und aus Methanol umkristallisiert. Man erhält 0,28 g (41 %) farblose Kristalle vom Schmp. 270 °C.
$C_{24}H_{25}N_8O$ (442,52)
$^1$H-NMR-Daten (DMSO-$d_6$, TMS als interner Standard)

δ = 1,1 (d) 3 H
1,9 - 2,2 (m) 2 H
2,3 - 3,1 (m) 4 H
3,2 - 3,6 (m) 3 H
4,5 (d) 2 H
7,3 - 8,0 (m) 9H,1H austauschbar mit $D_2O$
8,1 (t) 1 H,austauschbar mit $D_2O$
11,0 (s) 1 H,austauschbar mit $D_2O$ ppm

Beispiel 9

N[1]-Benzoyl-N[2]-methyl-N[3]-[3-[5-(4-methyl-6-oxo-1,4,5,6-tetrahydropyridazin 3 yl)-1H-benzimidazol-2-yl]-propyl]guanidin

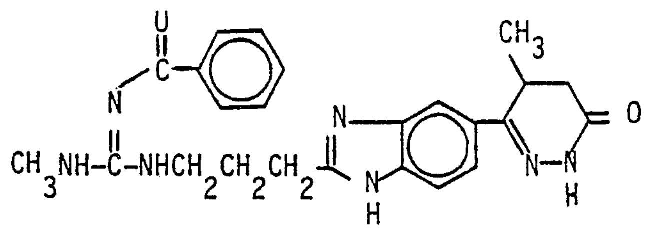

a) N[1]-Benzoyl-N[2]-[3-[5-(4 methyl-6-oxo-1,4,5,6-tetrahydropyridazin-3-yl)-1H-benzimidazol-2-yl]propyl]-O-phenyl-isoharnstoff

0,50 g (1,75 mmol) 6-[2-(3-Aminopropyl)-1H benzimidazol-5-yl] 4,5-dihydro-5-methyl-3(2H)-pyridazinon und 0,56 g (1.76 mmol) N-Benzoyl-diphenylimidocarbonat werden in 20 ml Isopropanol 3 h bei Raumtemperatur gerührt. Der ausgefallene Feststoff wird abgesaugt, mit etwas kaltem Isopropanol gewaschen und i. Vak. getrocknet. Man erhält 0,70 g (79 %) eines farblosen Feststoffs vom Schmp. 125 °C.
$C_{29}H_{28}N_6O_3$ (508,58)

b) N[1]-Benzoyl-N[2]-methyl-N[3]-[3-[5-(4-methyl-6-oxo-1,4,5,6-tetrahydropyridazin-3-yl)-IH-benzimidazol-2-yl]-propyl]guanidin

0.63 g (1.23 mmol) N[1]-Benzoyl-N[2]-[3-|5-(4-methyl-6-oxo-1,4,5,6-tetrahydropyridazin-3-yl)-1H-benzimidazol-2-yl]propyl]-O-phenyl-isoharnstoff in 20 ml Methanol werden mit 1,5 ml (16,5 mmol) einer Lösung von Methylamin in Methanol (11 Mol/l) versetzt und 4 Stunden gerührt. Das nach Abdampfen des Lösungmittels i.Vak. erhaltene gelbliche Öl wird an Kieselgel mit Dichlormethan/Methanol (9:1) als Laufmittel chromatographiert und ergibt nach Eindampfen der Hauptfraktion 0,48 g (87 %) eines farblosen. amorphen Feststoffs.
$C_{24}H_{27}N_7O_2$ (445,52)
$^1$H-NMR-Daten (DMSO-$d_6$, TMS als interner Standard)
δ = 1,15 (d) 3H
2,0 - 3,2 (m) 6 H
2,95 (s) 3 H
3,3 - 3,7 (m) 3 H
5,0 (breit) 4 H,austauschbar mit $D_2$
7,3 - 8,3 (m) 8 H ppm

Beispiel 10

$N^1$-Methyl-$N^2$-[3-[5-(4-methyl-6-oxo-1,4,5,6-tetrahydropyridazin--yl)-1H-benzimidazol-2-yl]propyl]-$N^3$-phenylsulfonyl-guanidin

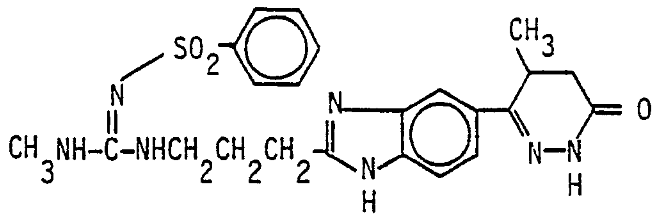

a) S-Methyl-$N^1$-[3-[5-(4-methyl-6-oxo-1,4,5,6-tetrahydropyridazin-3-yl-)-1H-benzimidazol-2-yl]propyl]-$N^2$-phenylsulfonyl-isoharnstoff

0,50 g (1,75 mmol) 6-[2-(3-Aminopropyl)-1H- benzimidazol-5-yl]-4,5-dihydro-5-methyl-3(2H)-pyridazinon und 0,56 g (1.75 mmol) N-Phenylsulfonyl-imin-dithiokohlensäuredimethylester werden in 20 ml Isopropanol 9 h bei 50 °C gerührt. Das Lösungsmittel wird i.Vak. abgedampft und der ölige Rückstand an Kieselgel mit Dichlormethan/Methanol (9:1) als Laufmittel chromatographiert. Nach Eindampfen der Hauptfraktion i.Vak. werden 0,85 g (97 %) eines gelblichen, amorphen Feststoffs erhalten.

$C_{23}H_{26}N_6O_3S_2$ (498,62)

$^1$H-NMR-Daten ($CDCl_3$,TMS als interner Standard)

$\delta$ = 1,2 (d) 3 H
2,0 - 2,7 (m) 4 H
2,3 (s) 3 H
2,8 - 3,1 (m) 2 H
3,2 - 3,6 (m) 3 H
7,4 - 8,2 (m) 8 H
8,5 (breit) 1 H,austauschbar mit $D_2O$
10,1 (breit) 1H,austauschbar mit $D_2O$ ppm

b) $N^1$-Methyl-$N^2$-[3-[5-(4-methyl-6-oxo-1,4,5,6-tetrahydropyridazin-3-yl)-1H-benzimidazol-2-yl]propyl]-$N^3$-phenylsulfonyl-guanidin

0.85 g (1,7 mmol) S-Methyl-$N^1$-[3-[5-(4-methyl-6-oxo-1,4,5,6-tetrahydropyridazin-3-yl)-1H-benzimidazol-2-yl]propyl]-$N^2$-phenylsulfonyl-isoharnstoff werden in 20 ml Ethanol mit 1,7 ml (17 mmol) einer Lösung von Methylamin in Methanol (10 Mol/l) bei Raumtemperatur gerührt. Nach 20 Stunden wird der ausgefallene Feststoff abgesaugt, mit Ethanol gewaschen und mit 30 ml Methanol ausgekocht. Der nach Absaugen erhaltene Feststoff wird i.Vak. getrocknet. Man erhält 0.65 g (79 %) eines farblosen Feststoffs vom Schmp. 258-260 °C.

$C_{23}H_{27}N_7O_3S$ (481,57)

$^1$H-NMR-Daten (DMSO-$d_6$, TMS als interner Standard)

$\delta$ = 1,15 (d) 3 H
1,9 - 3,1 (m) 6H
2,8 (d) 3 H
3,2 - 3,7 (m) 3 H
7,3 - 8,1 (m) 10H,2H austauschbar mit $D_2O$
11,1 (s) 1 H,austauschbar mit $D_2O$ ppm

Beispiel 11

6-[2-(2-Aminoethyl)-1H-benzimidazol-5-yl] 4,5 dihydro-5-methyl-3(2H)-pyridazinon

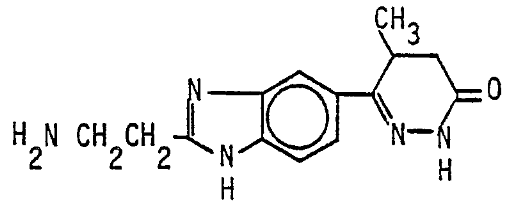

Hergestellt analog zu Beispiel 1d) aus 1,44 g (3,6 mmol) 6-|2-(2-Phthalimido ethyl)-1H-benzimidazol-5-yl]-4,5-dihydro-5-methyl-3(2H)-pyridazinon und 0,9 ml (18 mmol) Hydrazinhydrat. Das Rohprodukt wird an Kieselgel mit Methanol/konz. Ammoniak (99:1) chromatographiert. Nach Eindampfen der Hauptfraktion i.Vak. und Kristallisieren des Rückstands aus Ethanol erhält man 0,55 g (51 %) eines farblosen Feststoffs vom Schmp. 178 °C.

$C_{14}H_{17}N_5O$ (271,32)

$^1$H-NMR-Daten ($CD_3OD$,TMS als interner Standard)

$\delta$ = 1,2 (d) 3 H
2,2 - 2,9 (m) 2 H
3,1 - 3,3 (m) 4 H
3,3 - 3,7 (m) 1 H
5,4 (breit) 4H,austauschbar mit $D_2O$
7,6 - 8,2 (m) 3 H ppm

Beispiel 12

6-[2-(4-Aminobutyl)-1H-benzimidazol-5-yl]-4,5 dihydro-5-methyl-3(2H)-yridazinon

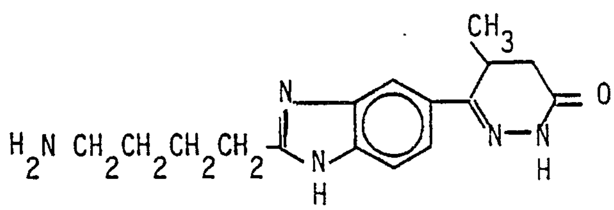

0,54 g (1,26 mmol) 6-[2-(4-Phthalimidobutyl)-1H-benzimidazol 5-yl]-4,5-dihydro-5-methyl-3(2H)-pyridazinon werden mit 0,3 ml (6,3 mmol) Hydrazinhydrat in 20 ml Ethanol 2 Stunden gekocht. Nach Abkühlen auf Raumtemperatur wird die Lösung filtriert, das Filtrat i.Vak. eingedampft und der erhaltene Rückstand in 10 ml 2 N Salzsäure aufgenommen. Nach erneuter Filtration wird die Lösung mit 3 N Natronlauge auf pH 11 gestellt und auf die Hälfte des ursprünglichen Volumens eingeengt. Beim Kühlen im Eisbad kristallisiert ein farbloser Feststoff, der aus Ethanol umkristallisiert wird. 0,26 g (69 %) Kristalle, die bei 185 - 187 °C schmelzen und wieder erstarren, und dann bis 280 °C nicht mehr schmelzen.

$C_{16}H_{21}N_5O$ (299,37)

$^1$H-NMR-Daten ($CD_3OD$,TMS als interner Standard)

$\delta$ = 1,2 (d) 3 H
1,5 - 2,1 (m) 4 H
2,3 - 3,2 (m) 6 H
3,3 - 3,7 (m) 1 H
4,9 (breit)4 H,austauschbar mit $D_2O$
7,5 - 8,2 (m) 3 H ppm

Beispiel 13

6-[2-(3-Aminopropyl)-1H-benzimidazol-5-yl]-4,5 dihydro-3(2H)-pyridazinon

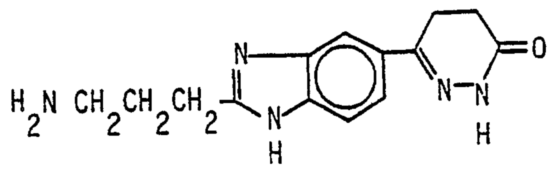

Hergestellt analog zu Beispiel 1d) aus 3,3 g (8,2 mmol) 6-[2-(3-Phthalimidopropyl)-1H-benzimidazol-5-yl]-4,5-dihydro-3(2H)-pyridazinon und 2,0 ml (41,1 mmol) Hydrazinhydrat. Nach chroamtographischer Reinigung am Kieselgel mit Methanol/konz. Ammoniak (95:5) als Laufmittel. Eindampfen der Hauptfraktion i.Vak. und Kristallisation des Rückstands aus Ethanol erhält man 0,77 g (35 %) eines farblosen Feststoffs vom Schmp. 247-249 °C (Zers.).

$C_{14}H_{17}N_5O$ (271,32)

$^1$H-NMR-Daten (DMSO-$d_6$,TMS als interner Standard)

δ = 1,7 - 2,1 (quin) 3 H
2,4 - 3,2 (m) 8 H
4,3 (breit) 4 H,austauschbar mit $D_2O$
7,5 - 8,1 (m) 3 H ppm

Beispiel 14

N[1]-[3-(Imidazol-4-yl)propyl]-N[2]-[4-[5-(4 methyl-6-tetrahydropyridazin-3-yl)-1H-benzimidazol-2 yl]butyl]-guanidin

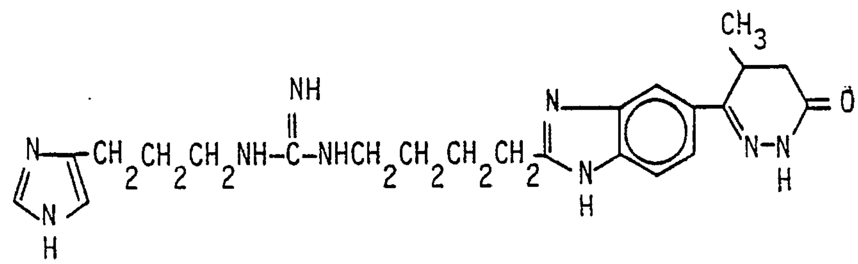

Aus 1,00 g (2,0 mmol) N-[4-[5-(4-Methyl-6-oxo-1,4,5,6- tetrahydropyridazin-3-yl)-1H-benzimidazol-2-yl]-butyl]-S-methyl-isothiuroniumjodid und 0,25 g (2,0 mmol) 3-(Imidazol-4-yl)propylamin erhält man analog zu Beispiel 6 0,48 g (53 %) eines farblosen, amorphen Feststoffs.

$C_{23}H_{31}N_9O$ (449,56)

$^1$H-NMR-Daten ($CD_3OD$, TMS als interner Standard)

δ = 1,2 (d) 3 H
1,6 - 3,7 (m) 17 H
5,0 (breit) 6 H, austauschbar mit $D_2O$
6,95 (s) 1 H
7,4 - 8,2 (m) 4 H ppm

Beispiel 15

6-[2-[3-(Ethoxycarbonylamino)propyl]-1H-benzimidazol-5-yl]-4,5-dihydro-5-methyl-3(2H)-pyridazinon

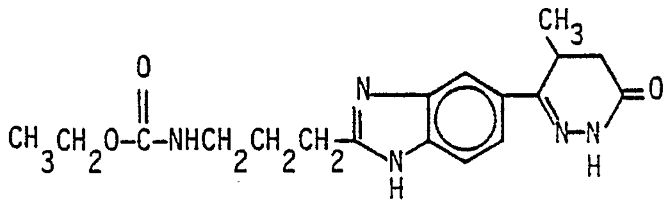

Zu einer Lösung von 1,00 g (3,5 mmol) 6-[2-(3-Aminopropyl)-1H-benzimidazol-5 yl]-4,5-dihydro-5-methyl-3(2H)-pyridazinon und 1,25 g (9,0 mmol) Kaliumcarbonat in 10 ml Aceton und 10 ml Wasser werden unter Kühlung im Eisbad 0,45 ml (4,7 mmol) Chlorameisensäureethylester während 10 Minuten zugetropft. Nach dreistündigem Rühren bei Raumtemperatur wird das Reatkionsgemisch filtriert, das Filtrat mit 20 ml Wasser verdünnt und dreimal mit 20 ml Dichlormethan extrahiert. Die vereinigten organischen Phasen werden nach Trocknen i. Vak. eingedampft und der Rückstand an Kieselgel mit Dichlormethan/Methanol (9:1) als Laufmittel chromatographiert. Die polare Hauptfraktion (Rf 0,46) ergibt nach Eindampfen i. Vak. 0,65 g (52 %) der Titelverbindung als gelblichen, amorphen Feststoff.

$C_{18}H_{23}N_5O_3$ (357,41)

$^1$H-NMR-Daten ($CD_3OD$, TMS als interner Standard)

$\delta$ = 1,1 - 1,3 (d+t) 6 H
1,8 - 2,2 (quin) 2 H
2,3 - 3,1 (m) 4 H
3,25 (t) 2 H
3,3 - 3,7 (m) 1 H
4,1 (q) 2 H
4,9 (breit) 3 H, austauschbar mit $D_2O$
7,5 - 8,1 (m) 3 H ppm

**Ansprüche**

1. Benzimidazole der allgemeinen Formel I

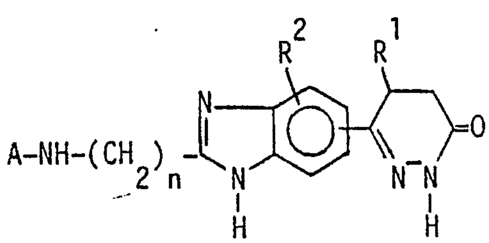

(I)

in denen der Pyridazinonring in 5- oder 6-Stellung des Benzimidazolrings gebunden ist und $R^1$ ein Wasserstoffatom oder eine gerad- oder verzweigtkettige $C_1$-$C_4$-Alkylgruppe bedeutet, $R^2$ für ein Wasserstoffatom, eine gerad- oder verzweigtkettige $C_1$-$C_4$-Alkylgruppe, eine $C_1$-$C_4$-Alkoxygruppe, eine Hydroxygruppe, ein Halogenatom, eine Aminogruppe oder eine Nitrogruppe steht, A für ein Wasserstoffatom, die Gruppe $-\underset{\underset{O}{\|}}{C}-R^3$,

$$-\overset{\overset{N-B}{\|}}{C}-NHR^3 \text{ oder } -\overset{\overset{CHNO_2}{\|}}{C}-NHR^3,$$

worin $R^3$ ein Wasserstoffatom, eine gegebenenfalls substituierte $C_1$-$C_4$-Alkylgruppe oder eine $C_1$-$C_4$-Alkoxygruppe und B eine Cyanogruppe, eine Benzoylgruppe oder eine Phenylsulfonylgruppe bedeuten, oder für die Gruppierung in der m den Wert 2 oder 3

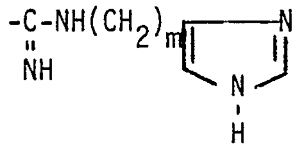 ,

hat, steht und n eine ganze Zahl von 1 bis 6 ist, sowie die physiologisch annehmbaren Salze davon.

2. Benzimidazole nach Anspruch 1, dadurch **gekennzeichnet,** daß $R^1$ für ein Wasserstoffatom oder eine gerad- oder verzweigtkettige $C_1$-$C_4$-Alkylgruppe, insbesondere eine Methylgruppe, steht, $R^2$ und A jeweils für ein Wasserstoffatom stehen und n eine ganze Zahl von 1 bis 6, inbesondere die Zahl 3, ist.

3. Benzimidazole nach Anspruch 1, dadurch **gekennzeichnet,** daß $R^1$ für ein Wasserstoffatom oder eine gerad- oder verzweigtkettige $C_1$-$C_4$-Alkylgruppe, insbesondere eine Methylgruppe, steht, $R^2$ ein Wasserstoffatom bedeutet, A für die Gruppe $-\underset{\underset{O}{\|}}{C}-R^3$ steht, wobei $R^3$ ein Wasserstoffatom, eine ggf. substituierte $C_1$-$C_4$-Alkylgruppe oder eine $C_1$-$C_4$-Alkoxygruppe ist, und n eine ganze Zahl von 1 bis 6, insbesondere die Zahl 3, ist.

4. Benzimidazole nach Anspruch 1, dadurch **gekennzeichnet,** daß $R^1$ für ein Wasserstoffatom oder eine gerad- oder verzweigtkettige $C_1$-$C_4$-Alkylgruppe, insbesondere eine Methylgruppe, steht, $R^2$ ein Wasserstoffatom ist, A für die Gruppe

$$\overset{N-B}{\overset{\|}{-C-NHR^3}}$$

steht, worin $R^3$ ein Wasserstoffatom oder eine gegebenenfalls substituierte $C_1$-$C_4$-Alkylgruppe ist und B eine Cyanogruppe, eine Benzoylgruppe oder eine Phenylsulfonylgruppe bedeutet, und n eine ganze Zahl von 1 bis 6, insbesondere die Zahl 3, ist.

5. Benzimidazole nach Anspruch 1, dadurch **gekennzeichnet,** daß $R^1$ für ein Wasserstoffatom oder eine gerad- oder verzweigtkettige $C_1$-$C_4$-Alkylgruppe, insbesondere eine Methylgruppe, und $R^2$ für ein Wasserstoffatom stehen, A die Gruppe

$$\overset{CHNO_2}{\overset{\|}{-C-NHR^3}}$$

bedeutet, worin $R^3$ für ein Wasserstoffatom oder eine gegebenenfalls substituierte $C_1$-$C_4$-Alkylgruppe steht, und n eine ganze Zahl von 1 bis 6, vorzugsweise die Zahl 3, ist.

6. Benzimidazole nach Anspruch 1, dadurch **gekennzeichnet,** daß $R^1$ für ein Wasserstoffatom oder eine gerad- oder verzweigtkettige $C_1$-$C_4$-Alkylgruppe, insbesondere eine Methylgruppe, steht, $R^2$ für ein Wasserstoffatom steht, A für die Gruppierung

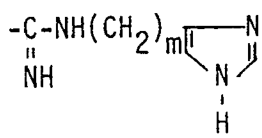

steht, in der m den Wert 2 oder 3 hat, und n eine ganze Zahl von 1 bis 6, insbesondere die Zahl 3, ist.

7. 6-[2-(3-Aminopropyl)-1H-benzimidazol-5-yl]-4,5-dihydro-5-methyl-3(2H)-pyridazinon und die physiologisch annehmbaren Salze.

8. $N^1$-[3-(Imidazol-4-yl)propyl]-$N^2$-[3-[5-(4-methyl-6-oxo-1,4,5,6-tetrahydropyridazin-3-yl)-1H-benzimidazol-2-yl]propyl]-guanidin und die physiologisch annehmbaren Salze.

9. 6-[2-(2-Aminoethyl)-1H-benzimidazol-5-yl]-4,5-dihydro-5-methyl-3(2H)-pyridazinon und die physiologisch annehmbaren Salze.

10. $N^1$-[3-(Imidazol-4-yl)propyl]-$N^2$-[2-[5-(4-methyl-6-oxo-1,4,5,6-tetrahydropyridazin-3-yl)-1H-benzimidazol-2-yl]ethyl]-guanidin und die physiologisch annehmbaren Salze.

11. 6-[2-(4-Aminobutyl)-1H-benzimidazol-5-yl]-4,5-dihydro-5-methyl-3(2H)-pyridazinon und die physiologisch annehmbaren Salze.

12. $N^1$-[3-(Imidazol-4-yl)propyl]-$N^2$-[4-[5-(4-methyl-6-oxo-1,4,5,6-tetrahydropyridazin-3-yl)-1H-benzimidazol-2-yl] butyl] -guanidin und die physiologisch annehmbaren Salze.

13. Verfahren zur Herstellung von Benzimidazolen der allgemeinen Formel I

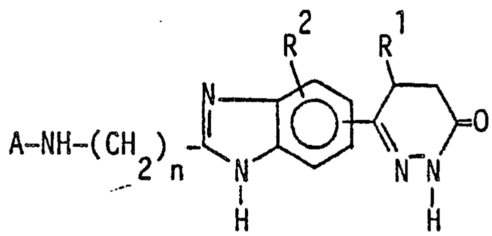

(I)

in denen der Pyridazinonring in 5- oder 6-Stellung des Benzimidazolrings gebunden ist und $R^1$ ein Wasserstoffatom oder eine gerad- oder verzweigtkettige $C_1$-$C_4$-Alkylgruppe bedeutet, $R^2$ für ein Wasserstoffatom, eine gerad- oder verzweigtkettige $C_1$-$C_4$-Alkylgruppe, eine $C_1$-$C_4$-Alkoxygruppe, eine Hydroxygruppe, ein Halogenatom, eine Aminogruppe oder eine Nitrogruppe steht, A für ein Wasserstoffatom, die Gruppe $-\underset{\underset{O}{\|}}{C}-R^3$,

$$\overset{N-B}{\overset{\|}{-C}}-NHR^3 \text{ oder } \overset{CHNO_2}{\overset{\|}{-C}}-NHR^3,$$

worin $R^3$ ein Wasserstoffatom, eine gegebenenfalls substituierte $C_1$-$C_4$-Alkylgruppe oder eine $C_1$-$C_4$-Alkoxygruppe und B eine Cyanogruppe, eine Benzoylgruppe oder eine Phenylsulfonylgruppe bedeuten, oder für die Gruppierung

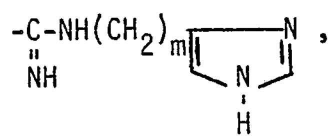

in der m den Wert 2 oder 3 hat, steht, und n eine ganze Zahl von 1 bis 6 ist, sowie der physiologisch annehmbaren Salze davon, dadurch **gekennzeichnet,** daß man

a) zur Herstellung von Verbindungen der allgemeinen Formel I, bei denen $R^1$, $R^2$ und n wie oben definiert sind und A für ein Wasserstoffatom steht,

$a_1$) von einer Phthalimidverbindung der allgemeinen Formel II

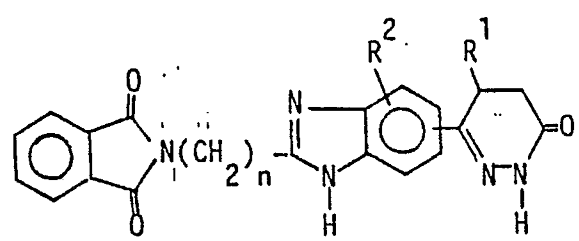

(II)

in der $R^1$, $R^2$ und n wie oben definiert sind, mit Hydrazin oder einem chemischen Äquivalent davon, einem aliphatischen primären Amin oder einer Säure die Phthalimidgruppe abspaltet und dadurch in eine Verbindung der allgemeinen Formel I überführt oder

$a_2$) eine Verbindung der allgemeinen Formel I

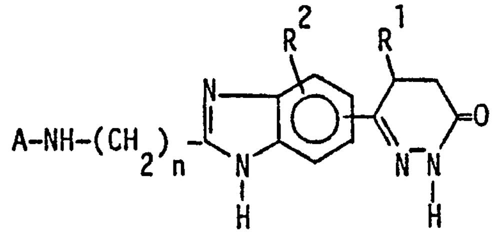

(I)

in der $R^1$, $R^2$ und n wie oben definiert sind und A für die Gruppe $-\underset{\underset{O}{\parallel}}{C}-R^3$ steht,

wobei $R^3$ wie oben definiert ist, sauer oder basisch hydrolysiert,

b) zur Herstellung von Verbindungen der allgemeinen Formel I, bei denen $R^1$, $R^2$ und n wie oben definiert sind und A für die Gruppe $-\underset{\underset{O}{\parallel}}{C}-R^3$ steht,

wobei $R^3$ die oben angegebene Definition hat,

eine Verbindung der allgemeinen Formel Ia

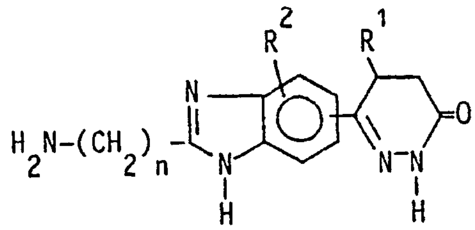

(Ia)

in der $R^1$, $R^2$ und n die oben angegebenen Bedeutungen besitzen und A für ein Wasserstoffatom steht, mit einem Acylierungsmittel der allgemeinen Formel III

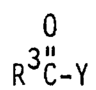

(III)

in der $R^3$ die oben angegebene Bedeutung hat und Y für ein Halogenatom, insbesondere ein Chlor- oder Bromatom, den Rest $-O-\underset{\underset{O}{\parallel}}{C}-R^3$,

wobei $R^3$ die oben angegebene Bedeutung hat, den über ein N-Atom gebundenen Rest eines Azols oder Benzazols mit mindestens 2 N-Atomen im Fünfring oder die Gruppe -OH steht, zu einer Verbindung der allgemeinen Formel I umsetzt oder

c) zur Herstellung von Verbindungen der allgemeinen Formel I, bei denen $R^1$, $R^2$ und n wie oben definiert sind und A für die Gruppe

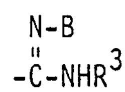

steht, worin $R^3$ und B wie oben definiert sind,

$c_1$) eine Verbindung der allgemeinen Formel Ia

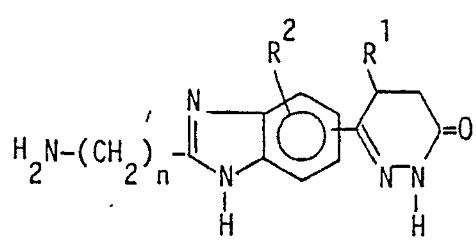

(Ia)

in der $R^1$, $R^2$ und n die oben angegebenen Bedeutungen haben, mit einer Verbindung der allgemeinen Formel IV

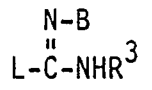

(IV)

in der B und $R^3$ wie oben definiert sind und L für eine Austrittsgruppe, zum Beispiel eine Alkylthio-, Arylthio-, Alkoxy- oder Aryloxygruppe, steht, zu einer Verbindung der allgemeinen Formel I umsetzt oder

$c_2$) eine Verbindung der allgemeinen Formel Ia

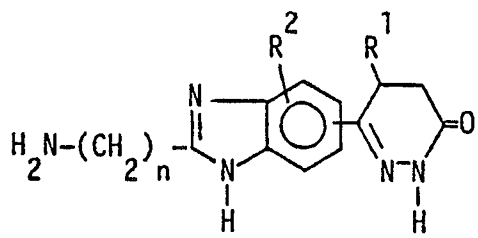

(Ia)

in der $R^1$, $R^2$ und n die oben angegebenen Bedeutungen haben, zunächst mit einer Verbindung der allgemeinen Formel V

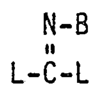

(V)

in der B und L die oben angegebenen Bedeutungen haben, zu einer Verbindung der allgemeinen Formel XIII

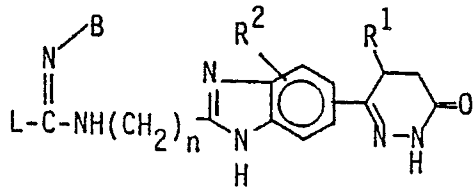

(XIII)

in der $R^1$, $R^2$, B, L und n die oben angegebenen Bedeutungen haben, umsetzt und diese sodann mit einer Verbindung der allgemeinen Formel VI

$R^3$-$NH_2$ (VI)

in der $R^3$ wie oben definiert ist, zu einer Verbindung der allgemeinen Formel I umsetzt oder

d) zur Herstellung von Verbindungen der allgemeinen Formel I, bei der $R^1$, $R^2$ und n wie oben definiert sind und A für die Gruppe

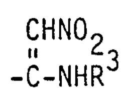

steht, in der $R^3$ die oben angegebene Bedeutung hat,

$d_1$) eine Verbindung der allgemeinen Formel Ia

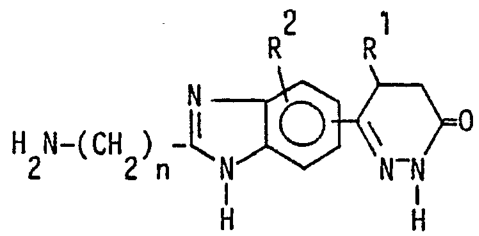

(Ia)

in der $R^1$, $R^2$ und n die oben angegebenen Bedeutungen haben, mit einer Verbindung der allgemeinen Formel VII

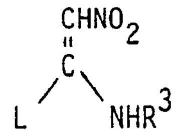

(VII)

in der L für eine Austrittsgruppe, zum Beispiel eine Alkylthio-, Arylthio-, Alkoxy- oder Aryloxygruppe, steht und $R^3$ die oben angegebene Bedeutung besitzt, zu einer Verbindung der allgemeinen Formel I umsetzt oder

$d_2$) eine Verbindung der allgemeinen Formel Ia

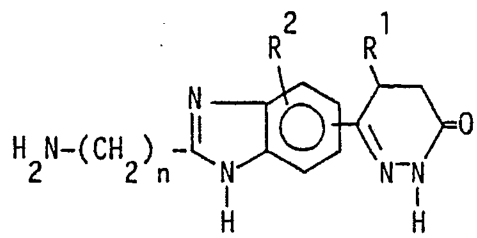

(Ia)

in der $R^1$, $R^2$ und n die oben angegebenen Bedeutungen haben, zunächst mit einer Verbindung der allgemeinen Formel VIII

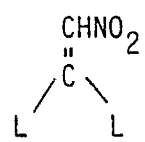

(VIII)

in der L die oben angegebene Bedeutung hat, zu einer Verbindung der allgemeinen Formel IX

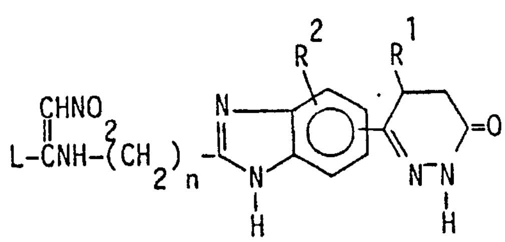

(IX)

in der $R^1$, $R^2$, n und L wie oben definiert sind, umsetzt und diese sodann mit einer Verbindung der allgemeinen Formel VI

$R^3$-$NH_2$ (VI)

in der $R^3$ wie oben definiert ist, zu einer Verbindung der allgemeinen Formel I umsetzt oder

e) zur Herstellung von Verbindungen der allgemeinen Formel I, bei denen $R^1$, $R^2$ und n wie oben definiert sind und A für die Gruppe

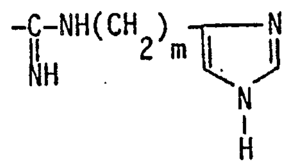

in der m den Wert 2 oder 3 hat, steht.

$e_1$) eine Verbindung der allgemeinen Formel X

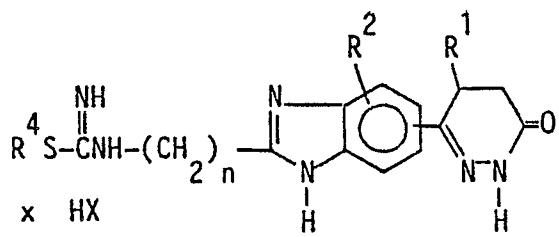

(X)

in der $R^1$, $R^2$ und n wie oben definiert sind, $R^4$ für eine gegebenenfalls substituierte $C_1$-$C_4$- Alkylgruppe oder eine Benzylgruppe steht und X für ein Halogenatom oder die Gruppe $-OSO_2OR^4$ steht, mit einem m-(Imidazol-4-yl)alkylamin der allgemeinen Formel XI

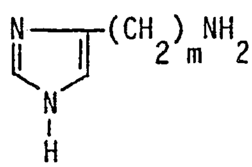

(XI)

in der m den Wert 2 oder 3 hat, zu einer Verbindung der allgemei nen Formel I umsetzt oder

$e_2$) eine Verbindung der allgemeinen Formel Ia

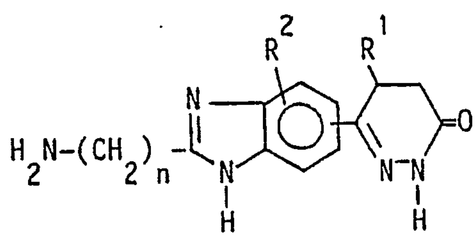

(Ia)

in der $R^1$, $R^2$ und n die oben angegebenen Bedeutungen haben, mit einer Verbindung der allgemeinen Formel XII

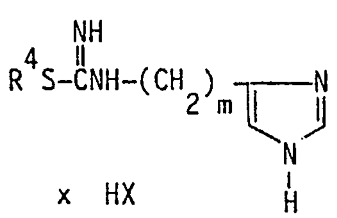

(XII)

in der m, $R^4$ und X wie oben definiert sind, zu einer Verbindung der allgemeinen Formel I umsetzt und gegebenenfalls die nach den Verfahrensvarianten a) bis e) erhaltenen Verbindungen der allgemeinen Formel I in an sich bekannter Weise in ein physiologisch annehmbares Salz umwandelt.

14. Arzneimittel, dadurch **gekennzeichnet,** daß es eine Verbindung nach einem der Ansprüche 1 bis 12 zusammen mit mindestens einem inerten, pharmazeutisch annehmbaren Träger oder Verdünnungsmittel enthält.

Patentansprüche für folgende Vertragsstaaten: GR, ES

1. Verfahren zur Herstellung von Benzimidazolen der allgemeinen Formel I

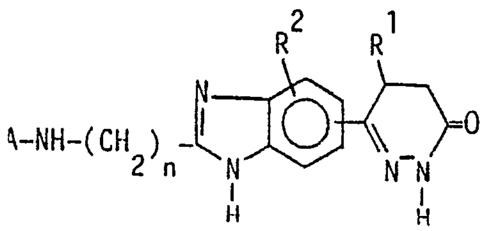

(I)

in denen der Pyridazinonring in 5- oder 6-Stellung des Benzimidazolrings gebunden ist und $R^1$ ein Wasserstoffatom oder eine gerad- oder verzweigtkettige $C_1$-$C_4$-Alkylgruppe bedeutet, $R^2$ für ein Wasserstoffatom, eine gerad- oder verzweigtkettige $C_1$-$C_4$-Alkylgruppe, eine $C_1$-$C_4$- Alkoxygruppe, eine Hydroxygruppe, ein Halogenatom, eine Aminogruppe oder eine Nitrogruppe steht, A für ein Wasserstoffatom, die Gruppe $-\underset{\underset{O}{\|}}{C}-R^3$,

$$-\overset{N-B}{\overset{\|}{C}}-NHR^3 \text{ oder } -\overset{CHNO_2}{\overset{\|}{C}}-NHR^3,$$

worin $R^3$ ein Wasserstoffatom, eine gegebenenfalls substituierte $C_1$-$C_4$- Alkylgruppe oder eine $C_1$-$C_4$-Alkoxygruppe und B eine Cyanogruppe, eine Benzoylgruppe oder eine Phenylsulfonylgruppe bedeuten, oder für die Gruppierung

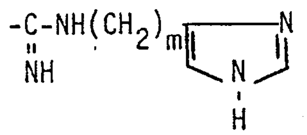

,

in der m den Wert 2 oder 3 hat, steht, und n eine ganze Zahl von 1 bis 6 ist, sowie der physiologisch annehmbaren Salze davon, dadurch **gekennzeichnet,** daß man

a) zur Herstellung von Verbindungen der allgemeinen Formel I, bei denen $R^1$, $R^2$ und n wie oben definiert sind und A für ein Wasserstoffatom steht,

$a_1$) von einer Phthalimidverbindung der allgemeinen Formel II

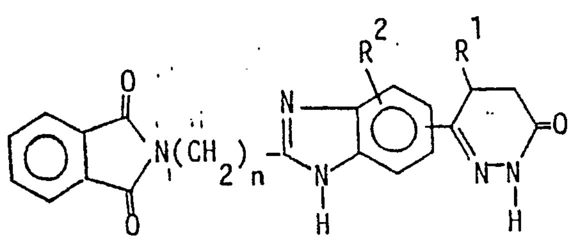

(II)

in der $R^1$, $R^2$ und n wie oben definiert sind, mit Hydrazin oder einem chemischen Äquivalent davon, einem aliphatischen primären Amin oder einer Säure die Phthalimidgruppe abspaltet und dadurch in eine Verbindung der allgemeinen Formel I überführt oder

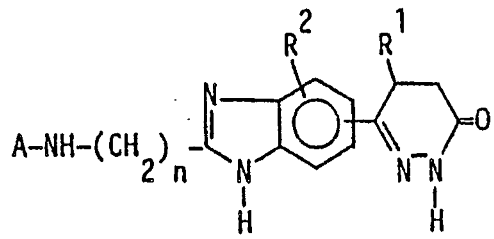

(I)

$a_2$) eine Verbindung der allgemeinen Formel I in der, $R^1$, $R^2$ und n wie oben definiert sind und A für die Gruppe $-\underset{\underset{O}{\|}}{C}-R^3$ steht,

wobei $R^3$ wie oben definiert ist, sauer oder basisch hydrolysiert,

b) zur Herstellung von Verbindungen der allgemeinen Formel I, bei denen $R^1$, $R^2$ und n wie oben definiert sind und A für die Gruppe $-\underset{\underset{O}{\|}}{C}-R^3$ steht,

wobei $R^3$ die oben angegebene Definition hat, eine Verbindung der allgemeinen Formel Ia

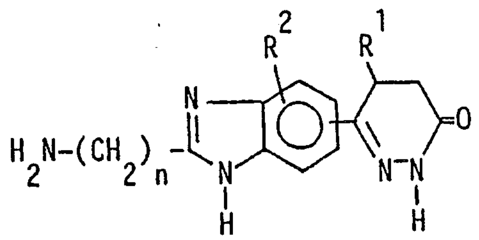

(Ia)

in der $R^1$, $R^2$ und n die oben angegebenen Bedeutungen besitzen und A für ein Wasserstoffatom steht, mit einem Acylierungsmittel der allgemeinen Formel III

$$R^3\overset{\overset{O}{\|}}{C}-Y \qquad (III)$$

in der $R^3$ die oben angegebene Bedeutung hat und Y für ein Halogenatom, insbesondere ein Chlor- oder Bromatom, den Rest $-O-\underset{\underset{O}{\|}}{C}-R^3$,

wobei $R^3$ die oben angegebene Bedeutung hat, den über ein N-Atom gebundenen Rest eines Azols oder Benzazols mit mindestens 2 N-Atomen im Fünfring oder die Gruppe -OH steht, zu einer Verbindung der allgemeinen Formel I umsetzt oder

c) zur Herstellung von Verbindungen der allgemeinen Formel I, bei denen $R^1$, $R^2$ und n wie oben definiert sind und A für die Gruppe N-B

$$-\overset{\overset{N-B}{\|}}{C}-NHR^3$$

steht, worin $R^3$ und B wie oben definiert sind,

$c_1$) eine Verbindung der allgemeinen Formel Ia

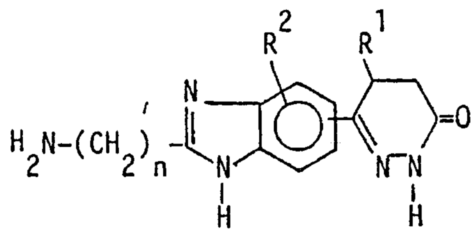

(Ia)

in der $R^1$, $R^2$ und n die oben angegebenen Bedeutungen haben, mit einer Verbindung der allgemeinen Formel IV

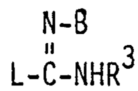

(IV)

in der B und $R^3$ wie oben definiert sind und L für eine Austrittsgruppe, zum Beispiel eine Alkylthio-, Arylthio-, Alkoxy- oder Aryloxygruppe, steht, zu einer Verbindung der allgemeinen Formel I umsetzt oder

$c_2$) eine Verbindung der allgemeinen Formel Ia

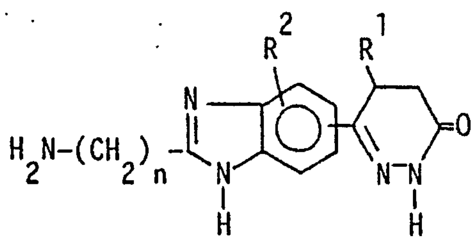

(Ia)

in der $R^1$, $R^2$ und n die oben angegebenen Bedeutungen haben, zunächst mit einer Verbindung der allgemeinen Formel V

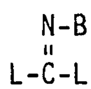

(V)

in der B und L die oben angegebenen Bedeutungen haben, zu einer Verbindung der allgemeinen Formel XIII

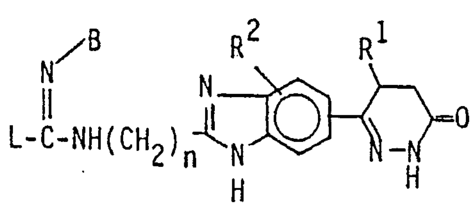

(XIII)

in der $R^1$, $R^2$, B, L und n die oben angegebenen Bedeutungen haben, umsetzt und diese sodann mit einer Verbindung der allgemeinen Formel VI

$R^3$-$NH_2$ (VI)

in der $R^3$ wie oben definiert ist, zu einer Verbindung der allgemeinen Formel I umsetzt oder

d) zur Herstellung von Verbindungen der allgemeinen Formel I, bei der $R^1$, $R^2$ und n wie oben definiert sind und A für die Gruppe

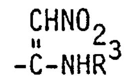

steht, in der $R^3$ die oben angegebene Bedeutung hat,
$d_1$) eine Verbindung der allgemeinen Formel Ia

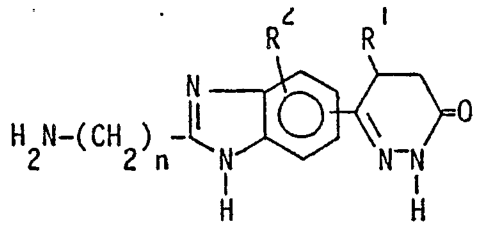
(Ia)

in der $R^1$, $R^2$ und n die oben angegebenen Bedeutungen haben, mit einer Verbindung der allgemeinen Formel VII

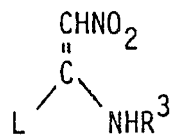
(VII)

in der L für eine Austrittsgruppe, zum Beispiel eine Alkylthio-, Arylthio-, Alkoxy- oder Aryloxygruppe, steht und $R^3$ die oben angegebene Bedeutung besitzt, zu einer Verbindung der allgemeinen Formel I umsetzt oder
$d_2$) eine Verbindung der allgemeinen Formel Ia

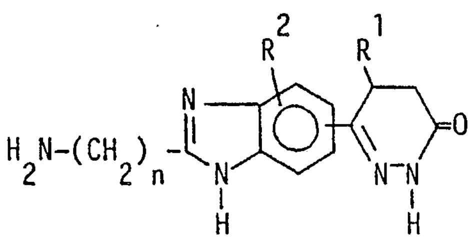
(Ia)

in der $R^1$, $R^2$ und n die oben angegebenen Bedeutungen haben, zunächst mit einer Verbindung der allgemeinen Formel VIII

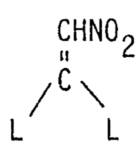
(VIII)

in der L die oben angegebene Bedeutung hat, zu einer Verbindung der allgemeinen Formel IX

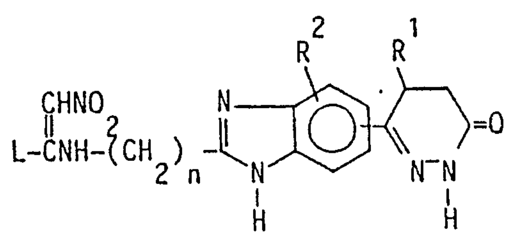
(IX)

in der R', $R^2$, n und L wie oben definiert sind, umsetzt und diese sodann mit einer Verbindung der

allgemeinen Formel VI

$R^3$-$NH_2$ (VI)

in der $R^3$ wie oben definiert ist, zu einer Verbindung der allgemeinen Formel I umsetzt oder

e) zur Herstellung von Verbindungen der allgemeinen Formel I, bei denen $R^1$, $R^2$ und n wie oben definiert sind und A für die Gruppe

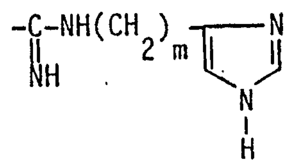

in der m den Wert 2 oder 3 hat, steht,

$e_1$) eine Verbindung der allgemeinen Formel X

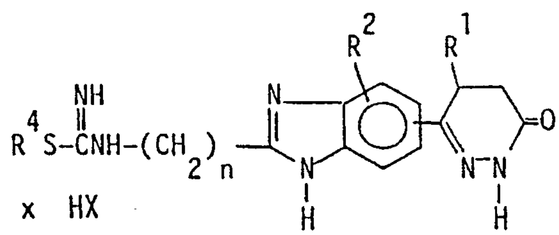

(X)

in der $R^1$, $R^2$ und n wie oben definiert sind, $R^4$ für eine gegebenenfalls substituierte $C_1$-$C_4$- Alkylgruppe oder eine Benzylgruppe steht und X für ein Halogenatom oder die Gruppe -$OSO_2OR^4$ steht, mit einem m-(Imidazol-4-yl)alkylamin der allgemeinen Formel XI

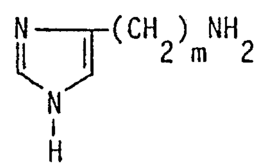

(XI)

in der m den Wert 2 oder 3 hat, zu einer Verbindung der allgemei nen Formel I umsetzt oder

$e_2$) eine Verbindung der allgemeinen Formel Ia

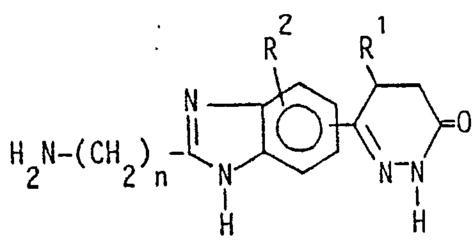

(Ia)

in der $R^1$, $R^2$ und n die oben angegebenen Bedeutungen haben, mit einer Verbindung der allgemeinen Formel XII

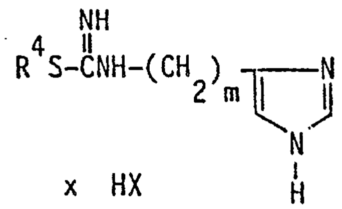

(XII)

in der m, $R^4$ und X wie oben definiert sind, zu einer Verbindung der allgemeinen Formel I umsetzt und gegebenenfalls die nach den Verfahrensvarianten a) bis e) erhaltenen Verbindungen der allgemeinen Formel I in an sich bekannter Weise in ein physiologisch annehmbares Salz umwandelt.

2. Verfahren nach Anspruch 1 zur Herstellung von Verbindungen, bei denen $R^1$ für ein Wasserstoffatom oder eine gerad- oder verzweigtkettige $C_1$-$C_4$ Alkylgruppe, insbesondere eine Methylgruppe, steht, $R^2$ und A jeweils für ein Wasserstoffatom stehen und n eine ganze Zahl von 1 bis 6, insbesondere die Zahl 3, ist.

3. Verfahren nach Anspruch 1 zur Herstellung von Verbindungen, bei denen $R^1$ für ein Wasserstoffatom oder eine gerad- oder verzweigtkettige $C_1$-$C_4$-Alkylgruppe, insbesondere eine Methylgruppe, steht, $R^2$ ein Wasserstoffatom bedeutet, A für die Gruppe $-\underset{\overset{\|}{O}}{C}-R^3$ steht,

wobei $R^3$ ein Wasserstoffatom, eine gegebenenfalls substituierte $C_1$-$C_4$-Alkylgruppe oder eine $C_1$-C-Alkoxygruppe ist, und n eine ganze Zahl von 1 bis 6, insbesondere die Zahl 3, ist.

4. Verfahren nach Anspruch 1 zur Herstellung von Verbindungen, bei denen $R^1$ für ein Wasserstoffatom oder eine gerad- oder verzweigtkettige $C_1$-$C_4$-Alkylgruppe, insbesondere eine Methylgruppe, steht, $R^2$ ein Wasserstoffatom ist, A für die Gruppe

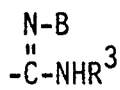

steht, worin $R^3$ ein Wasserstoffatom oder eine gegebenenfalls substituierte $C_1$-$C_4$-Alkylgruppe ist und B eine Cyanogruppe, eine Benzoylgruppe oder eine Phenylsulfonylgruppe bedeutet, und n eine ganze Zahl von 1 bis 6, insbesondere die Zahl 3, ist.

5. Verfahren nach Anspruch 1 zur Herstellung von Verbindungen, bei denen $R^1$ für ein Wasserstoffatom oder eine gerad- oder verzweigtkettige $C_1$-$C_4$-Alkylgruppe, insbesondere eine Methylgruppe, und $R^2$ für ein Wasserstoffatom stehen, A die Gruppe

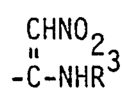

bedeutet, worin $R^3$ für ein Wasserstoffatom oder eine gegebenenfalls substituierte $C_1$-$C_4$ Alkylgruppe steht, und n eine ganze Zahl von 1 bis 6, vorzugsweise die Zahl 3, ist.

6. Verfahren nach Anspruch 1 zur Herstellung von Verbindungen, bei denen $R^1$ für ein Wasserstoffatom oder eine gerad- oder verzweigtkettige $C_1$-$C_4$-Alkylgruppe, insbesondere eine Methylgruppe, steht, $R^2$ für ein Wasserstoffatom steht, A für die Gruppierung

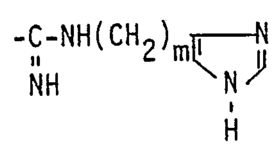

steht, in der m den Wert 2 oder 3 hat, und n eine ganze Zahl von 1 bis 6, insbesondere die Zahl 3, ist.

7. Verfahren nach Anspruch 1 zur Herstellung von 6-[2-(3-Aminopropyl)-1H-benzimidazol-5-yl]-4,5-dihydro-5-methyl-3(2H)-pyridazinon und der physiologisch annehmbaren Salze.

8. Verfahren nach Anspruch 1 zur Herstellung von $N^1$-[3-(Imidazol-4-yl)propyl]-$N^2$-[3-[5-(4-methyl-6-oxo-1,4,5,6-tetrahydropyridazin-3-yl)-1H-benzimidazol-2-yl]propyl]-guanidin und der physiologisch annehmbaren Salze.

9. Verfahren nach Anspruch 1 zur Herstellung von 6-[2-Aminoethyl)1H-benzimidazol-5-yl]-4,5-dihydro-5-methyl-3(2H)-pyridazinon und der physiologisch annehmbaren Salze.

10. Verfahren nach Anspruch 1 zur Herstellung von $N^1$-[3-(Imidazol-4-yl)propyl]-$N^2$-[2-[5-(4-methyl-6-oxo-1,4,5,6-tetrahydropyridazin-3-yl)-1H-benzimidazol-2-yl]ethyl]-guanidin und der physiologisch annehmbaren Salze.

11. Verfahren nach Anspruch 1 zur Herstellung von 6-[2-(4-Aminobutyl)-1H-benzimidazol-5-yl]-4,5-dihydro-5-methyl-3(2H)-pyridazinon und der physiologisch annehmbaren Salze.

12. Verfahren nach Anspruch 1 zur Herstellung von $N^1$-[3-(Imidazol-4-yl)propyl]-$N^2$-[4-[5-(4-methyl-6-oxo-1,4,5,6-tetrahydropyridazin-3-yl)-1H-benzimidazol-2-yl]butyl]-guanidin und der physiologisch annehmbaren Salze.

Europäisches Patentamt

# EUROPÄISCHER RECHERCHENBERICHT

Nummer der Anmeldung

EP 88 10 2619

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| --- | --- | --- | --- |
| A | EP-A-0 196 005 (K. THOMAE)<br>--- | | C 07 D 403/04<br>A 61 K 31/415<br>A 61 K 31/50 |
| A | EP-A-0 193 013 (MERCK)<br>--- | | |
| A | EP-A-0 125 749 (WARNER-LAMBERT)<br>--- | | |
| A | WO-A-8 703 201 (RORER INTERNATIONAL (OVERSEAS))<br>----- | | |
| | | | **RECHERCHIERTE SACHGEBIETE (Int. Cl.4)** |
| | | | C 07 D 403/00<br>A 61 K 31/00 |

Der vorliegende Recherchenbericht wurde für alle Patentansprüche erstellt

| Recherchenort | Abschlußdatum der Recherche | Prüfer |
| --- | --- | --- |
| DEN HAAG | 22-12-1988 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)